(19) Europäisches Patentamt
European Patent Office
Office européen des brevets

(11) Veröffentlichungsnummer: **0 009 569**
**B1**

(12) # EUROPÄISCHE PATENTSCHRIFT

(45) Veröffentlichungstag der Patentschrift: 28.04.82

(21) Anmeldenummer: 79102817.8

(22) Anmeldetag: 06.08.79

(51) Int. Cl.³: C 07 C 103/48, C 07 C 121/34, C 07 C 149/243, C 07 C 153/07, A 01 N 37/30

(54) **N-Oxalyl-Derivate von N-Phenyl-aminosäure(n) (estern), Verfahren zu ihrer Herstellung sowie ihre Verwendung als Fungizide.**

(30) Priorität: 18.08.78 DE 2836158

(43) Veröffentlichungstag der Anmeldung: 16.04.80 Patentblatt 80/8

(45) Bekanntmachung des Hinweises auf die Patenterteilung: 28.04.82 Patentblatt 82/17

(84) Benannte Vertragsstaaten: AT BE CH DE FR GB IT NL

(56) Entgegenhaltungen:
DE - A1 - 2 648 074
DE - A1 - 2 819 878
US - A - 3 780 090

Research Disclosure, Januar 1979, "Herbicidal compositions", *S. 44, Spalte 2*

(73) Patentinhaber: BAYER AG
Zentralbereich Patente, Marken und Lizenzen
D-5090 Leverkusen 1, Bayerwerk (DE)

(72) Erfinder: Lunkenheimer, Winfried, Dr.
Bismarckstrasse 29
D-5600 Wuppertal 1 (DE)
Erfinder: Brandes, Wilhelm, Dr.
Eichendorffstrasse 3
D-5653 Leichlingen (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 009 569 B1

N-Oxalyl-Derivate von N-Phenyl-aminosäure(n) (estern), Verfahren zu ihrer Herstellung sowie ihre Verwendung als Fungizide

Die vorliegende Erfindung betrifft neue N-Oxalyl-Derivate von N-Phenyl-aminosäure(n) (estern), mehrere Verfahren zu ihrer Herstellung und ihre Verwendung als Fungizide.

Es ist bereits bekannt geworden, daß Zink-äthylen-1,2-bisdithiocarbamidat ein gutes Mittel zur Bekämpfung von pilzlichen Pflanzenkrankheiten ist (vergleiche Phytopathology *33*, 1113 (1963)). Jedoch ist dessen Einsatz nur beschränkt möglich, da es bei niedrigen Aufwandmengen und -konzentrationen, insbesondere auch bei der Bekämpfung von Phytophthora-Arten, nicht immer eine zufriedenstellende Wirkung zeigt.

Aus der DE—AS 26 48 074 sind N-Chloracetyl-N-phenylalaminester und ihre Verwendung als Fungizide bekannt. Jedoch müssen diese in relativ hohen Konzentrationen angewendet werden, um eine befriedigende fungizide Wirksamkeit zu zeigen.

Es wurden neue N-Oxalyl-Derivate von N-Phenyl-aminosäure(n) (estern) der allgemeinen Formel

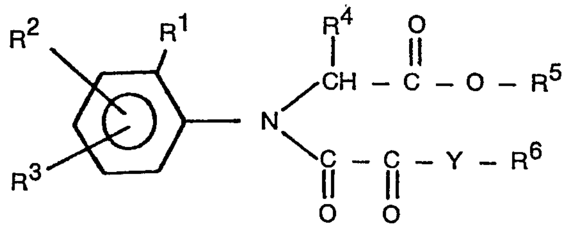

(I)

in welcher

$R^1$ für Wasserstoff, Alkyl oder Halogen steht,

$R^2$ für Wasserstoff oder Alkyl steht,

$R^3$ für Wasserstoff oder Alkyl steht,

$R^4$ für Wasserstoff, Alkyl oder gegebenenfalls substituiertes Phenyl steht,

$R^5$ für Wasserstoff, Alkyl, Cyanalkyl, Alkenyl, Alkinyl, Halogenalkyl, Cycloalkyl, Cycloalkylalkyl, Alkoxyalkyl, Alkoxyalkoxyalkyl, Alkoxycarbonylalkyl, Alkylthioalkyl, Alkylsulfinylalkyl, Alkylsulfonylalkyl, Dialkylaminoalkyl oder gegebenenfalls substituiertes Aryl und Aralkyl steht,

$R^6$ für Alkyl, Cyanalkyl, Alkenyl, Alkinyl, Halogenalkyl, Cycloalkyl, Cycloalkylalkyl, Alkoxyalkyl, Alkoxyalkoxyalkyl, Alkoxycarbonylalkyl, Alkylthioalkyl, Alkylsulfinylalkyl, Alkylsulfonylalkyl, Dialkylaminoalkyl, gegebenenfalls substituiertes Aryl, Aralkyl oder Aroxyalkyl, Epoxialkyl sowie die Gruppierungen

$$[—X—\overset{\oplus}{N}R^7R^8R^9]\ Z^{\ominus} \quad \text{und} \quad —X—\overset{\overset{O}{\uparrow}}{N}R^7R^8$$

steht, wobei

$R^7$, $R^8$ und $R^9$ gleich oder verschieden sind und für Alkyl stehen,

X für Alkylen oder Alkyliden steht,

Y für Sauerstoff oder Schwefel steht und

Z für das Anion einer anorganischen oder organischen Säure steht,

gefunden. Sie weisen starke fungizide Eigenschaften auf.

Man erhält die N-Oxalyl-Derivate von N-Phenyl-aminosäure(n) (estern) der Formel (I), indem man N-Phenylaminosäure(n) (ester) der Formel

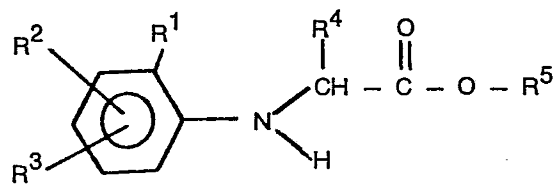

(II)

in welcher

$R^1$ bis $R^5$ die oben angegebene Bedeutung haben,

a) mit Chlorglyoxylsäureestern der Formel

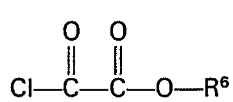

(III)

in welcher

$R^6$ die oben angegebene Bedeutung hat, in Gegenwart eines Lösungsmittels und gegebenenfalls in Gegenwart eines Säurebindemittels und/oder in Gegenwart eines Katalysators umsetzt, oder

b) zunächst mit Oxalylchlorid der Formel

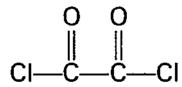

(IV)

in Gegenwart eines Lösungsmittels und gegebenenfalls in Gegenwart eines Säurebindemittels und/oder in Gegenwart eines Katalysators umsetzt und anschließend die dabei entstehenden Verbindungen der Formel

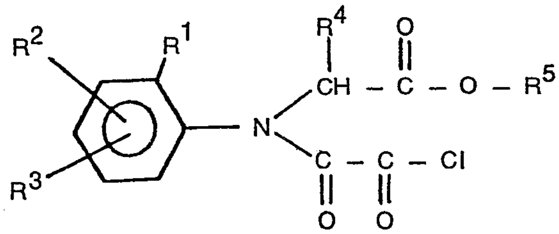

(V)

in welcher
$R^1$ bis $R^5$ die oben angegebene Bedeutung haben,
mit Alkoholen bzw. Mercaptanen der Formel

$$H-Y-R^6 \qquad (VI)$$

in welcher
$R^6$ und Y die oben angegebene Bedeutung haben,
in Gegenwart eines Lösungsmittels und gegebenefalls in Gegenwart eines Säurebindemittels und/oder in Gegenwart eines Katalysators umsetzt.

Ueberraschenderweise zeigen die erfindungsgemäßen N-Oxalyl-Derivate von N-Phenyl-aminosäure(n) (estern) eine erheblich höhere fungizide Wirksamkeit, insbesondere gegenüber Phytophthora-Arten, als das aus dem Stand der Technik bekannte Zink-äthlen-1,2-bisdithiocarbamidat, welches eine Verbindung gleicher Wirkungsrichtung ist.

Die erfindungsgemäßen Stoffe stellen somit eine Bereicherung der Technik dar.

Von den erfindungsgemäßen Verbindungen der Formel (I) sind bevorzugt diejenigen, in denen $R^1$ für Wasserstoff, geradkettiges oder verzweigtes Alkyl mit 1 bis 4 Kohlenstoffatomen und Halogen, insbesondere Fluor, Chlor oder Brom steht; $R^2$ für Wasserstoff oder geradkettiges oder verzweigtes Alkyl mit 1 bis 4 Kohlenstoffatomen steht; $R^3$ für Wasserstoff oder geradkettiges oder verzweigtes Alkyl mit 1—4 Kohlenstoffatomen steht; $R^4$ für Wasserstoff, geradkettiges oder verzweigtes Alkyl mit 1 bis 4 Kohlenstoffatomen und gegebenenfalls substituiertes Phenyl steht, wobei als Substituenten vorzugsweise infrage kommen: Halogen, insbesondere Fluor, Chlor oder Brom, Alkyl mit 1 bis 2 Kohlenstoffatomen sowie Halogenalkyl mit bis zu 2 Kohlenstoff- und bis zu 3 gleichen oder verschiedenen Halogenatomen, wobei als Halogene vorzugsweise Fluor und Chlor stehen; $R^5$ und $R^6$ für geradkettiges oder verzweigtes Alkyl und Cyanalkyl mit jeweils 1 bis 6 Kohlenstoffatomen, Alkenyl und Alkinyl mit jeweils 2 bis 4 Kohlenstoffatomen; für Halogenalkyl mit 1 bis 4 Kohlenstoff- und bis zu 5 Halogenatomen, insbesondere mit bis zu 2 Kohlenstoff- und bis zu 3 gleichen oder verschiedenen Halogenatomen, wobei als Halogene vorzugsweise Fluor und Chlor stehen; für Cycloalkyl und Cycloalkylalkyl mit jeweils 3 bis 7 Kohlenstoffatomen im Cycloalkylteil und 1 bis 4 Kohlenstoffatomen im Alkylteil; für Alkoxyalkyl, Alkoxyalkoxyalkyl, Alkoxycarbonylalkyl, Alkylthioalkyl, Alkylsulfinylalkyl und Alkylsulfonylalkyl mit jeweils 1 bis 4 Kohlenstoffatomen in jedem Alkylteil; sowie für gegebenenfalls im Arylteil substituiertes Aryl oder Aralkyl mit 6 bis 10 Kohlenstoffatomen im Arylteil und 1 bis 4 Kohlenstoffatomen im Alkylteil, wie insbesondere Phenyl oder Benzyl, wobei als Substituenten vorzugsweise die bei $R^4$ bereits genannten infrage kommen, und für Dialkylaminoalkyl mit 1 bis 4 Kohlenstoffatomen in jedem Alkylteil stehen; $R^5$ außerdem für Wasserstoff steht; $R^6$ außerdem für gegebenenfalls im Arylteil substituiertes Aroxyalkyl mit 6 bis 10 Kohlenstoffatomen im Arylteil und 1 bis 4 Kohlenstoffatomen im Alkylteil steht, wobei als Substituenten vorzugsweise die bei $R^4$ bereits genannten infrage kommen; Epoxyialkyl mit insgesamt 3 bis 8 Kohlenstoffatomen sowie für die Gruppen

$$[-X-\overset{\oplus}{N}R^7R^8R^9]\,Z^{\ominus} \quad \text{und} \quad -X-\overset{\overset{O}{\uparrow}}{N}R^7R^8$$

steht, $R^7$, $R^8$ und $R^9$ gleich oder verschieden sind und für Alkyl mit 1 bis 4 Kohlenstoffatomen stehen; $X$ für Alkylen mit 1 bis 4 Kohlenstoffatomen und Alkyliden mit 2 bis 4 Kohlenstoffatomen steht; $Y$ für Sauerstoff oder Schwefel steht und $Z$ für das Anion einer anorganischen Säure steht.

Ganz besonders bevorzugt sind diejenigen Verbindungen der Formel (I) in denen $R^1$, $R^2$ und $R^3$ für

Wasserstoff, Methyl, Ethyl, n-Propyl, Isopropyl, n-Butyl, Isobutyl, sek-Butyl oder tert.-Butyl stehen; $R^1$ außerdem für Fluor, Chlor oder Brom steht; $R^4$ für Wasserstoff, Methyl, gegebenenfalls durch Chlor, Methyl und/oder Ethyl substituiertes Phenyl steht; $R^5$ und $R^6$ für Methyl, Ethyl, n-Propyl, Isopropyl, n-Butyl, Isobutyl, sek.-Butyl, tert.-Butyl, Cyanethyl, Vinyl, Allyl, Propargyl, Chlorethyl, Cyclopropyl, Cyclopropylmethyl, Cyclohexyl, Cyclohexylmethyl, Methoxyethyl, Ethoxyethyl, Methylthioethyl, Ethylthioethyl, Methoxyethoxyethyl, Butoxycarbonylmethyl, Methylsulfinylethyl, Ethylsulfinylethyl, Methylsulfonylethyl, Ethylsulfonylethyl, gegebenenfalls durch Chlor, Methyl und/oder Ethyl substituiertes Phenyl oder Benzyl sowie Dimethylaminoethyl oder Diethylaminoethyl stehen; $R^5$ außerdem für Wasserstoff steht; $R^6$ außerdem für Phenoxyethyl steht, wobei der Phenylteil gegebenenfalls durch Methyl und/oder Ethyl substituiert sein kann, weiterhin für Ethylenoxidmethyl, Ethylenoxidethyl sowie für die Gruppen

$$[-X-\overset{\oplus}{N}R^7R^8R^9]\ Z^{\ominus} \text{ und } -X-\overset{\overset{O}{\uparrow}}{N}R^7R^8$$

steht, wobei $R^7$, $R^8$ und $R^9$ gleich oder verschieden sind und für Methyl oder Ethyl stehen und $X$ für Methylen, Ethylen, Ethyliden oder Propyliden steht; $Y$ für Sauerstoff oder Schwefel steht und $Z$ für ein Halogenid-Anion, wie Chlorid, Bromid oder Jodid steht, ferner für Nitrat-, Sulfat-, Phosphat- sowie Acetat-, Propionat-, Glykolat-, Lactat-, Malonat-, Tartrat-, Benzoat-, Methansulfonat-, p-Toluolsulfonat-, Benzolsulfonat- oder Methylsulfat-Anionen steht.

Im Einzelnen seien außer den bei den Herstellungsbeispielen genannten Verbindungen noch die folgenden Verbindungen der allgemeinen Formel (I) genannt:

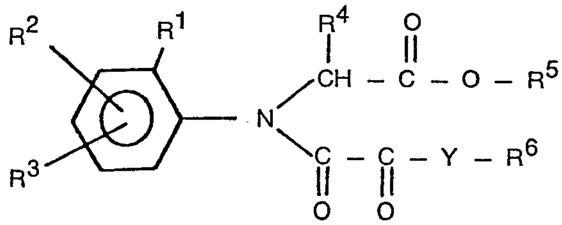

| $R^1$ | $R^2$ | $R^3$ | $R^4$ | $R^5$ | $R^6$ | Y |
|---|---|---|---|---|---|---|
| $CH_3$ | H | 6-$CH_3$ | $CH_3$ | $CH_3$ | n-$C_3H_7$ | O |
| $CH_3$ | H | 6-$CH_3$ | $CH_3$ | $CH_3$ | t-$C_4H_9$ | O |
| $CH_3$ | H | 6-$CH_3$ | $CH_3$ | $CH_3$ | $-CH_2CCl_3$ | O |
| $CH_3$ | H | 6-$CH_3$ | $CH_3$ | $CH_3$ | $-CH_2$ 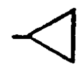 | O |
| $CH_3$ | H | 6-$CH_3$ | $CH_3$ | $CH_3$ | $-CH_2$ 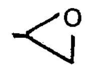 | O |
| $CH_3$ | H | 6-$CH_3$ | $CH_3$ | $CH_3$ | $-CH_2-CH_2-SO-C_2H_5$ | O |
| $CH_3$ | H | 6-$CH_3$ | $CH_3$ | $CH_3$ | $-CH_2-CH_2-SO_2-C_2H_5$ | O |
| $CH_3$ | H | 6-$CH_3$ | $CH_3$ | $CH_3$ | $-CH_2-CH_2\overset{\oplus}{N}(CH_3)_3$ $CH_3OSO_3^{\ominus}$ | O |
| $CH_3$ | H | 6-$CH_3$ | $CH_3$ | $CH_3$ | $-CH_2-CH_2-N(CH_3)_2$ 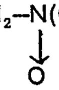 | O |
| $CH_3$ | H | 6-$CH_3$ | $CH_3$ | $CH_3$ | 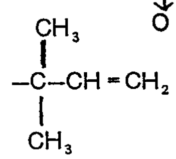 | O |
| $CH_3$ | H | 6-$CH_3$ | $CH_3$ | $CH_3$ | 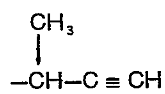 | O |
| $CH_3$ | H | 6-$CH_3$ | $CH_3$ | $CH_3$ | 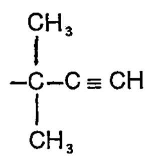 | O |
| $CH_3$ | H | 6-$CH_3$ | $CH_3$ | $CH_3$ | $CH_3$ | S |
| $CH_3$ | H | 6-$CH_3$ | $CH_3$ | $CH_3$ | $C_2H_5$ | S |

Verwendet man beispielsweise N-(2,6-Xylyl)-alaninmethylester und Methoxalylchlorid als Ausgangsstoffe, so kann der Reaktionsblauf durch das folgende Formelschema wiedergegeben werden (Verfahren a):

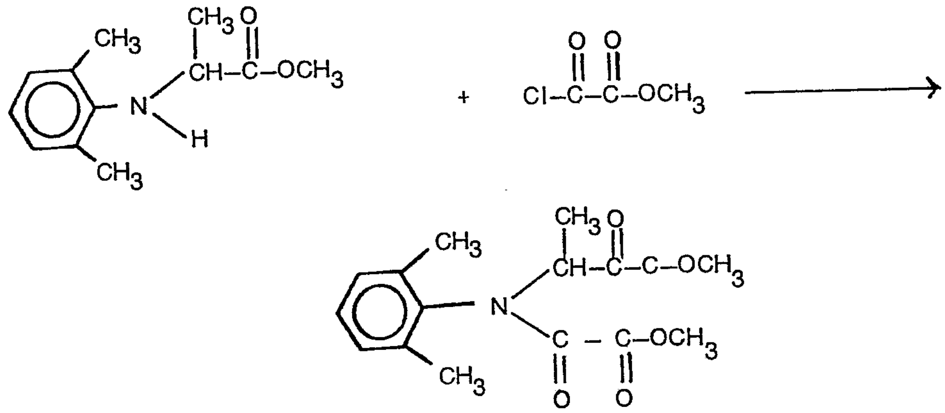

Verwendet man N-(2,6-Xylyl)-alaninmethylester, Oxalylchlorid und Methylglykol als Ausgangsstoffe, so kann der Reaktionsablauf durch das folgende Formelschema wiedergegeben werden (Verfahren b):

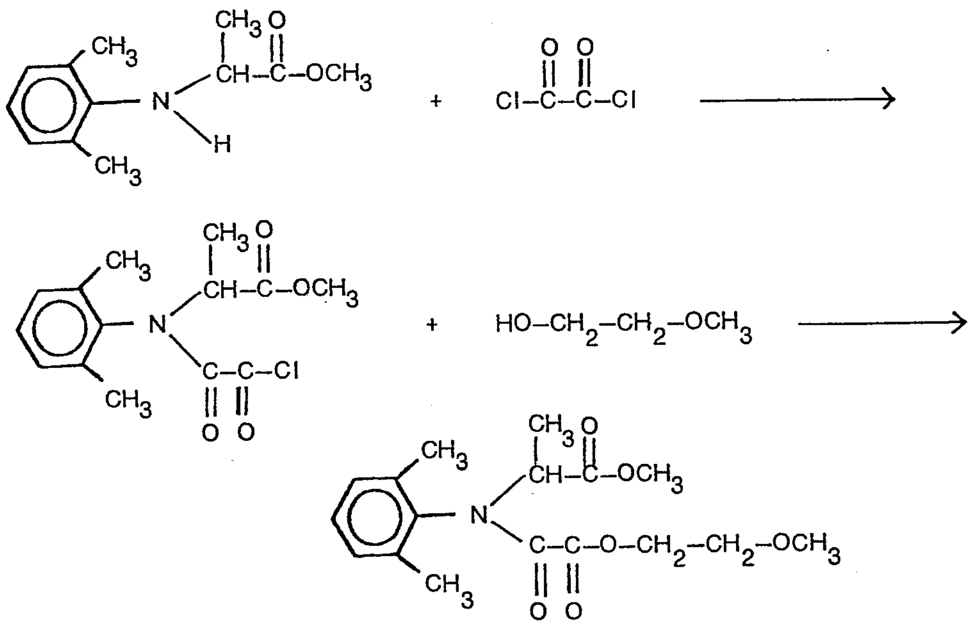

Die als Ausgangsstoffe zu verwendenden N-Phenyl-aminosäure(n) (ester) sind durch die Formel (II) allgemein definiert. In dieser Formel stehen $R^1$ bis $R^5$ vorzugsweise für die Reste, die bei den Verbindungen der Formel (I) bereits vorzugsweise genannt wurden.

Die N-Phenyl-aminosäure(n) (ester) der Formel (II) sind bekannt (vergleiche u.a. die Deutsche Offenlegungsschrift 2 648 074 (Le A 17 487) sowie die US-Patentschrift 3 780 090). Noch nicht bekannte können nach den dort beschriebenen Verfahren erhalten werden, indem man z.B. entsprechende Aniline mit entsprechenden $\alpha$-Halogencarbonsäure(n) (estern), wie beispielsweise Chloressigsäure(estern) oder $\alpha$-Chlorpropionsäure(estern), in Gegenwart eines Säurebinders, wie beispielsweise Kaliumcarbonat, und in Gegenwart eines inerten organischen Lösungsmittels, wie beispielsweise Dimethylformamid, und gegebenenfalls in Gegenwart eines Katalysators, wie z.B. Kaliumjodid, bei Temperaturen zwischen 20 und 160°C umsetzt. Die N-Phenyl-aminosäureester der Formel (II) können auch erhalten werden, indem man N-Phenyl-aminosäuren mit den entsprechenden Alkoholen nach bekannten Verfahren, z.B. in Gegenwart von Bortrifluorid, verestert.

Als Beispiele für die Ausgangsstoffe der Formel (II) seien genannt:

N-(2,6-Dimethylphenyl)-alanin(glycin)
N-(2,6-Dimethylphenyl)-alanin(glycin)-methylester
N-(2,6-Dimethylphenyl)-alanin(glycin)-ethylester
N-(2,6-Dimethylphenyl)-alanin(glycin)-isopropylester
N-(2,6-Dimethylphenyl)-alanin(glycin)-2-methoxyethylester
N-(2,6-Dimethylphenyl)-alanin(glycin)-2-ethoxyethylester

N-(2,6-Dimethylphenyl)-alanin(glycin)-cyclohexylester
N-(2,6-Dimethylphenyl)-alanin(glycin)-cyclohexylmethylester
N-(2,6-Dimethylphenyl)-alanin(glycin)-cyclopropylmethylester
N-(2,6-Dimethylphenyl)-alanin(glycin)-2-methylthioethylester
N-(2,6-Dimethylphenyl)-alanin(glycin)-2-methylsulfinylethylester
N-(2,6-Dimethylphenyl)-alanin(glycin)-2-methylsulfonylethylester
N-(2,6-Dimethylphenyl)-alanin(glycin)-2-dimethylaminoethylester
N-(2,6-Dimethylphenyl)-alanin(glycin)-benzylester
N-(2,6-Dimethylphenyl)-alanin(glycin)-2,4-dichlorbenzylester
N-(2,6-Dimethylphenyl)-alanin(glycin)-2-(2-methoxyethoxy)ethylester
N-(2,6-Dimethylphenyl)-alanin(glycin)-2-cyanmethylester
N-(2,6-Dimethylphenyl)-alanin(glycin)-phenylester
N-(2,6-Dimethylphenyl)-alanin(glycin)-allylester
N-(2,6-Dimethylphenyl)-alanin(glycin)-propargylester
N-(2,6-Dimethylphenyl)-alanin(glycin)-chlorethylester
N-(2,6-Dimethylphenyl)-alanin(glycin)-2,2,2-trichlorethylester
N-(2,6-Dimethylphenyl)-alanin(glycin)-2-ethylthioethylester
N-(2,6-Dimethylphenyl)-alanin(glycin)-2-ethylsulfinylethylester
N-(2,6-Dimethylphenyl)-alanin(glycin)-2-ethylsulfonylethylester
N-(2,6-Dimethylphenyl)-alanin(glycin)-butoxycarbonylmethylester
N-(2,6-Dimethylphenyl)-alanin(glycin)-1-ethoxycarbonylethylester
N-(2-Ethyl-6-methylphenyl)-alanin(glycin)
N-(2-Ethyl-6-methylphenyl)-alanin(glycin)-methylester
N-(2-Ethyl-6-methylphenyl)-alanin(glycin)-ethylester
N-(2-Ethyl-6-methylphenyl)-alanin(glycin)-isopropylester
N-(2-Ethyl-6-methylphenyl)-alanin(glycin)-2-methoxyethylester
N-(2-Ethyl-6-methylphenyl)-alanin(glycin)-2-ethoxyethylester
N-(2-Ethyl-6-methylphenyl)-alanin(glycin)-cyclohexylester
N-(2-Ethyl-6-methylphenyl)-alanin(glycin)-cyclohexylmethylester
N-(2-Ethyl-6-methylphenyl)-alanin(glycin)-cyclopropylmethylester
N-(2-Ethyl-6-methylphenyl)-alanin(glycin)-2-methylthioethylester
N-(2-Ethyl-6-methylphenyl)-alanin(glycin)-2-methylsulfinylethylester
N-(2-Ethyl-6-methylphenyl)-alanin(glycin)-2-methylsulfonylethylester
N-(2-Ethyl-6-methylphenyl)-alanin(glycin)-2-dimethylaminoethylester
N-(2-Ethyl-6-methylphenyl)-alanin(glycin)-benzylester
N-(2-Ethyl-6-methylphenyl)-alanin(glycin)-2,4-dichlorbenzylester
N-(2-Ethyl-6-methylphenyl)-alanin(glycin)-2-(2-methoxyethoxy)-ethylester
N-(2-Ethyl-6-methylphenyl)-alanin(glycin)-2-cyanethylester
N-(2-Ethyl-6-methylphenyl)-alanin(glycin)-phenylester
N-(2-Ethyl-6-methylphenyl)-alanin(glycin)-allylester
N-(2-Ethyl-6-methylphenyl)-alanin(glycin)-propargylester
N-(2-Ethyl-6-methylphenyl)-alanin(glycin)-2-chlorethylester
N-(2-Ethyl-6-methylphenyl)-alanin(glycin)-2,2,2-trichlorethylester
N-(2-Ethyl-6-methylphenyl)-alanin(glycin)-2-ethylthioethylester
N-(2-Ethyl-6-methylphenyl)-alanin(glycin)-2-ethylsulfinylethylester
N-(2-Ethyl-6-methylphenyl)-alanin(glycin)-2-ethylsulfonylethylester
N-(2-Ethyl-6-methylphenyl)-alanin(glycin)-2-butoxycarbonylmethylester
N-(2-Ethyl-6-methylphenyl)-alanin(glycin)-1-ethoxycarbonylethylester
N-(2-Chlor-6-methylphenyl)-alanin(glycin)
N-(2-Chlor-6-methylphenyl)-alanin(glycin)-methylester
N-(2-Chlor-6-methylphenyl)-alanin(glycin)-ethylester
N-(2-Chlor-6-methylphenyl)-alanin(glycin)-2-methoxyethylester
N-(2-Chlor-6-methylphenyl)-alanin(glycin)-2-ethoxyethylester
N-(2-Chlor-6-methylphenyl)-alanin(glycin)-2-methylthioethylester
N-(2-Chlor-6-methylphenyl)-alanin(glycin)-2-dimethylaminoethylester
N-(2-Chlor-6-methylphenyl)-alanin(glycin)-2-cyanethylester
N-(2-Chlor-6-methylphenyl)-alanin(glycin)-allylester
N-(2-Chlor-6-methylphenyl)-alanin(glycin)-propargylester
N-(2-Chlor-6-methylphenyl)-alanin(glycin)-2,2,2-trichlorethylester
N-(2-Chlor-6-methylphenyl)-alanin(glycin)-1-ethoxycarbonylethylester
N-(2-Chlor-6-ethylphenyl)-alanin(glycin)
N-(2-Chlor-6-ethylphenyl)-alanin(glycin)-methylester
N-(2-Chlor-6-ethylphenyl)-alanin(glycin)-ethylester
N-(2-Chlor-6-ethylphenyl)-alanin(glycin)-2-methoxyethylester
N-(2-Chlor-6-ethylphenyl)-alanin(glycin)-2-methylthioethylester

N-(2-Chlor-6-ethylphenyl)-alanin(glycin)-2-dimethylaminoethylester
N-(2-Chlor-6-ethylphenyl)-alanin(glycin)-2-cyanethylester
N-(2-Chlor-6-ethylphenyl)-alanin(glycin)-allylester
N-(2-Chlor-6-ethylphenyl)-alanin(glycin)-propargylester
N-(2-Chlor-6-ethylphenyl)-alanin(glycin)-2,2,2-trichlorethylester
N-(2-Chlor-6-ethylphenyl)-alanin(glycin)-1-ethoxycarbonylethylester
N-(2,4,6-Trimethylphenyl)-alanin(glycin)
N-(2,4,6-Trimethylphenyl)-alanin(glycin)-methylester
N-(2,4,6-Trimethylphenyl)-alanin(glycin)-ethylester
N-(2,4,6-Trimethylphenyl)-alanin(glycin)-2-methoxyethylester
N-(2,4,6-Trimethylphenyl)-alanin(glycin)-2-methylthioethylester
N-(2,4,6-Trimethylphenyl)-alanin(glycin)-2-dimethylaminoethylester
N-(2,4,6-Trimethylphenyl)-alanin(glycin)-2-cyanethylester
N-(2,4,6-Trimethylphenyl)-alanin(glycin)-allylester
N-(2,4,6-Trimethylphenyl)-alanin(glycin)-propargylester
N-(2,4,6-Trimethylphenyl)-alanin(glycin)-2,2,2-trichlorethylester
N-(2,4,6-Trimethylphenyl)-alanin(glycin)-1-ethoxycarbonylethylester
N-(2-Ethyl-4,6-dimethylphenyl)-alanin(glycin)
N-(2-Ethyl-4,6-dimethylphenyl)-alanin(glycin)-methylester
N-(2-Ethyl-4,6-dimethylphenyl)-alanin(glycin)-ethylester
N-(2-Ethyl-4,6-dimethylphenyl)-alanin(glycin)-methoxyethylester
N-(2-Ethyl-4,6-dimethylphenyl)-alanin(glycin)-methylthioethylester
N-(2-Ethyl-4,6-dimethylphenyl)-alanin(glycin)-dimethylaminoethylester
N-(2-Ethyl-4,6-dimethylphenyl)-alanin(glycin)-2-cyanethylester
N-(2-Ethyl-4,6-dimethylphenyl)-alanin(glycin)-allylester
N-(2-Ethyl-4,6-dimethylphenyl)-alanin(glycin)-propargylester
N-(2-Ethyl-4,6-dimethylphenyl)-alanin(glycin)-2,2,2-trichlorethylester
N-(2-Ethyl-4,6-dimethylphenyl)-alanin(glycin)-1-ethoxycarbonylethylester
N-(2,6-Diethyl-4-methylphenyl)-alanin(glycin)
N-(2,6-Diethyl-4-methylphenyl)-alanin(glycin)-methylester
N-(2,6-Diethyl-4-methylphenyl)-alanin(glycin)-2-methoxyethylester
N-(2,6-Diethyl-4-methylphenyl)-alanin(glycin)-2-methylthioethylester
N-(2,6-Diethyl-4-methylphenyl)-alanin(glycin)-2-dimethylaminoethylester
N-(2,6-Diethyl-4-methylphenyl)-alanin(glycin)-2-cyanethylester
N-(2,6-Diethyl-4-methylphenyl)-alanin(glycin)-allylester
N-(2,6-Diethyl-4-methylphenyl)-alanin(glycin)-propargylester
N-(2,6-Diethyl-4-methylphenyl)-alanin(glycin)-2,2,2-trichlorethylester
N-(2,6-Diethyl-4-methylphenyl)-alanin(glycin)-1-ethoxycarbonylethylester
N-(2,6-Diisopropylphenyl)-alanin(glycin)
N-(2,6-Diisopropylphenyl)-alanin(glycin)-methylester
N-(2,6-Diisopropylphenyl)-alanin(glycin)-ethylester
N-(2,6-Diisopropylphenyl)-alanin(glycin)-methoxyethylester
N-(2,6-Diisopropylphenyl)-alanin(glycin)-methylthioethylester
N-(2,6-Diisopropylphenyl)-alanin(glycin)-2-dimethylaminoethylester
N-(2,6-Diisopropylphenyl)-alanin(glycin)-2-cyanethylester
N-(2,6-Diisopropylphenyl)-alanin(glycin)-allylester
N-(2,6-Diisopropylphenyl)-alanin(glycin)-propargylester
N-(2,6-Diisopropylphenyl)-alanin(glycin)-2,2,2-trichlorethylester
N-(2,6-Diisopropylphenyl)-alanin(glycin)-1-ethoxycarbonylethylester
N-(2-Methylphenyl)-alanin(glycin)
N-(2-Methylphenyl)-alanin(glycin)-methylester
N-(2-Methylphenyl)-alanin(glycin)-ethylester
N-(2-Methylphenyl)-alanin(glycin)-2-methoxyethylester
N-(2-Methylphenyl)-alanin(glycin)-2-methylthioethylester
N-(2-Methylphenyl)-alanin(glycin)-2-dimethylaminoethylester
N-(2-Methylphenyl)-alanin(glycin)-2-cyanethylester
N-(2-Methylphenyl)-alanin(glycin)-allylester
N-(2-Methylphenyl)-alanin(glycin)-propargylester
N-(2-Methylphenyl)-alanin(glycin)-2,2,2-trichlorethylester
N-(2-Methylphenyl)-alanin(glycin)-1-ethoxycarbonylethylester
N-(2-Ethylphenyl)-alanin(glycin)
N-(2-Ethylphenyl)-alanin(glycin)-methylester
N-(2-Ethylphenyl)-alanin(glycin)-ethylester
N-(2-Ethylphenyl)-alanin(glycin)-2-methoxyethylester
N-(2-Ethylphenyl)-alanin(glycin)-2-methylthioethylester

N-(2-Ethylphenyl)-alanin(glycin)-2-cyanethylester
N-(2-Ethylphenyl)-alanin(glycin)-allylester
N-(2-Ethylphenyl)-alanin(glycin)-propargylester
N-(2-Ethylphenyl)-alanin(glycin)-2,2,2-trichlorethylester
N-(2-Ethylphenyl)-alanin(glycin)-1-ethoxycarbonylethylester
N-(2,3-Dimethylphenyl)-alanin(glycin)
N-(2,3-Dimethylphenyl)-alanin(glycin)-methylester
N-(2,3-Dimethylphenyl)-alanin(glycin)-ethylester
N-(2,3-Dimethylphenyl)-alanin(glycin)-2-methoxyethylester
N-(2,3-Dimethylphenyl)-alanin(glycin)-2-methylthioethylester
N-(2,3-Dimethylphenyl)-alanin(glycin)-2-dimethylaminoethylester
N-(2,3-Dimethylphenyl)-alanin(glycin)-2-cyanethylester
N-(2,3-Dimethylphenyl)-alanin(glycin)-allylester
N-(2,3-Dimethylphenyl)-alanin(glycin)-propargylester
N-(2,3-Dimethylphenyl)-alanin(glycin)-2,2,2-trichlorethylester
N-(2,3-Dimethylphenyl)-alanin(glycin)-1-ethoxycarbonylethylester
N-(2,5-Dimethylphenyl)-alanin(glycin)
N-(2,5-Dimethylphenyl)-alanin(glycin)-methylester
N-(2,5-Dimethylphenyl)-alanin(glycin)-ethylester
N-(2,5-Dimethylphenyl)-alanin(glycin)-2-methoxyethylester
N-(2,5-Dimethylphenyl)-alanin(glycin)-2-methylthioethylester
N-(2,5-Dimethylphenyl)-alanin(glycin)-2-dimethylaminoethylester
N-(2,5-Dimethylphenyl)-alanin(glycin)-2-cyanethylester
N-(2,5-Dimethylphenyl)-alanin(glycin)-allylester
N-(2,5-Dimethylphenyl)-alanin(glycin)-propargylester
N-(2,5-Dimethylphenyl)-alanin(glycin)-2,2,2-trichlorethylester
N-(2,5-Dimethylphenyl)-alanin(glycin)-1-ethoxycarbonylethylester
N-(2,4-Dimethylphenyl)-alanin(glycin)
N-(2,4-Dimethylphenyl)-alanin(glycin)-methylester
N-(2,4-Dimethylphenyl)-alanin(glycin)-ethylester
N-(2,4-Dimethylphenyl)-alanin(glycin)-2-methoxyethylester
N-(2,4-Dimethylphenyl)-alanin(glycin)-2-methylthioethylester
N-(2,4-Dimethylphenyl)-alanin(glycin)-2-dimethylaminoethylester
N-(2,4-Dimethylphenyl)-alanin(glycin)-2-cyanethylester
N-(2,4-Dimethylphenyl)-alanin(glycin)-allylester
N-(2,4-Dimethylphenyl)-alanin(glycin)-propargylester
N-(2,4-Dimethylphenyl)-alanin(glycin)-2,2,2-trichlorethylester
N-(2,4-Dimethylphenyl)-alanin(glycin)-1-ethoxycarbonylethylester
N-(2-Isopropylphenyl)-alanin(glycin)
N-(2-Isopropylphenyl)-alanin(glycin)-methylester
N-(2-Isopropylphenyl)-alanin(glycin)-ethylester
N-(2-Isopropylphenyl)-alanin(glycin)-2-methoxyethylester
N-(2-Isopropylphenyl)-alanin(glycin)-2-methylthioethylester
N-(2-Isopropylphenyl)-alanin(glycin)-2-dimethylaminoethylester
N-(2-Isopropylphenyl)-alanin(glycin)-2-cyanethylester
N-(2-Isopropylphenyl)-alanin(glycin)-allylester
N-(2-Isopropylphenyl)-alanin(glycin)-propargylester
N-(2-Isopropylphenyl)-alanin(glycin)-2,2,2-trichlorethylester
N-(2-Isopropylphenyl)-alanin(glycin)-1-ethoxycarbonylethylester
N-(2,4,5-Trimethylphenyl)-alanin(glycin)
N-(2,4,5-Trimethylphenyl)-alanin(glycin)-methylester
N-(2,4,5-Trimethylphenyl)-alanin(glycin)-ethylester
N-(2,4,5-Trimethylphenyl)-alanin(glycin)-2-methoxyethylester
N-(2,4,5-Trimethylphenyl)-alanin(glycin)-2-methylthioethylester
N-(2,4,5-Trimethylphenyl)-alanin(glycin)-2-dimethylaminoethylester
N-(2,4,5-Trimethylphenyl)-alanin(glycin)-2-cyanethylester
N-(2,4,5-Trimethylphenyl)-alanin(glycin)-allylester
N-(2,4,5-Trimethylphenyl)-alanin(glycin)-propargylester
N-(2,4,5-Trimethylphenyl)-alanin(glycin)-2,2,2-trichlorethylester
N-(2,4,5-Trimethylphenyl)-alanin(glycin)-1-ethoxycarbonylethylester
N-(2,3,5-Trimethylphenyl)-alanin(glycin)
N-(2,3,5-Trimethylphenyl)-alanin(glycin)-methylester
N-(2,3,5-Trimethylphenyl)-alanin(glycin)-ethylester
N-(2,3,5-Trimethylphenyl)-alanin(glycin)-2-methoxyethylester
N-(2,3,5-Trimethylphenyl)-alanin(glycin)-2-methylthioethylester

N-(2,3,5-Trimethylphenyl)-alanin(glycin)-2-dimethylaminoethylester
N-(2,3,5-Trimethylphenyl)-alanin(glycin)-2-cyanethylester
N-(2,3,5-Trimethylphenyl)-alanin(glycin)-allyl-ester
N-(2,3,5-Trimethylphenyl)-alanin(glycin)-propargylester
N-(2,3,5-Trimethylphenyl)-alanin(glycin)-2,2,2-trichlorethylester
N-(2,3,5-Trimethylphenyl)-alanin(glycin)-1-ethoxycarbonylethylester
N-(2,3,6-Trimethylphenyl)-alanin(glycin)
N-(2,3,6-Trimethylphenyl)-alanin(glycin)-methylester
N-(2,3,6-Trimethylphenyl)-alanin(glycin)-ethylester
N-(2,3,6-Trimethylphenyl)-alanin(glycin)-2-methoxyethylester
N-(2,3,6-Trimethylphenyl)-alanin(glycin)-2-dimethylaminoethylester
N-(2,3,6-Trimethylphenyl)-alanin(glycin)-2-cyanethylester
N-(2,3,6-Trimethylphenyl)-alanin(glycin)-allylester
N-(2,3,6-Trimethylphenyl)-alanin(glycin)-propargylester
N-(2,3,6-Trimethylphenyl)-alanin(glycin)-2,2,2-trichlorethylester
N-(2,3,6-Trimethylphenyl)-alanin(glycin)-1-ethoxycarbonylethylester
N-(2-sek.-Butylphenyl)-alanin(glycin)
N-(2-sek.-Butylphenyl)-alanin(glycin)-methylester
N-(2-sek.-Butylphenyl)-alanin(glycin)-ethylester
N-(2-sek.-Butylphenyl)-alanin(glycin)-2-methoxyethylester
N-(2-sek.-Butylphenyl)-alanin(glycin)-2-methylthioethylester
N-(2-sek.-Butylphenyl)-alanin(glycin)-2-dimethylaminoethylester
N-(2-sek.-Butylphenyl)-alanin(glycin)-2-cyanoethylester
N-(2-sek.-Butylphenyl)-alanin(glycin)-allylester
N-(2-sek.-Butylphenyl)-alanin(glycin)-propargylester
N-(2-sek.-Butylphenyl)-alanin(glycin)-2,2,2-trichlorethylester
N-(2-sek.-Butylphenyl)-alanin(glycin)-1-ethoxycarbonylethylester.

Die außerdem für die Verfahrensvariante (a) als Ausgangsstoffe zu verwendenden Chlorglyoxylsäureester sind durch die Formel (III) allgemein definiert. In dieser Formel steht $R^6$ vorzugsweise für die Reste, die bei den Verbindungen der Formel (I) bereits vorzugsweise genannt wurden.

Die Chlorglyoxylsäureester der Formel (III) sind teilweise bekannt, oder können durch Umsetzung von Oxalylchlorid mit Alkoholen nach bekannten Verfahren (vgl. G. v. Frank und W. Caro, Ber.dtsch.chem.Ges. *63*, 1532 (1930); S. J. Rhoads und R. E. Michel, J.Am.chem.Soc. *85*, 585 (1963)) hergestellt werden.

Als Beispiele seien genannt: Methoxalylchlorid, Ethoxalylchlorid, Isopropoxalylchlorid, Phenoxalychlorid, Methylphenoxalylchlorid, Dimethylphenoxalylchlorid, Diethylphenoxalylchlorid, Methoxyethoxyalylchlorid, Methoxyethoxyethoxyalylchlorid, Cyanethoxalylchlorid, Ethylthioethoxalylchlorid, Phenoxyethoxalylchlorid, Allyloxalylchlorid, Propargyloxalylchlorid, Chlorethoxalylchlorid, Cyclohexyloxalylchlorid, Benoxalylchlorid.

Das weiterhin für die Verfahrensvariante (b) als Ausgangsstoff zu verwendende Oxalylchlorid ist durch die Formel (IV) definiert. Oxalylchlorid ist eine allgemein bekannte Verbindung der organischen Chemie.

Die außerdem für die Verfahrensvariante (b) als Ausgangsstoffe zu verwendenden Alkohole bzw. Mercaptane sind durch die Formel (VI) allgemein definiert. In dieser Formel stehen $R^6$ und $Y$ vorzugsweise für die Reste, die bei den Verbindungen der Formel (I) bereits vorzugsweise genannt wurden.

Die Alkohole bzw. Mercaptane der Formel (VI) sind allgemein bekannte Verbindungen der organischen Chemie. Als Beispiele seien genannt: Methylalkohol, Ethylalkohol, Ispropylalkohol, Methylmercaptan, Butylmercaptan, Methylglykol, O-Methyldiethylenglykol, 2-Cyanethanol, Glykolsäurebutylester, 2-Ethylthioethanol, 2-Phenoxyethanol, 2-Dimethylaminoethanol, Propargylalkohol, Allylalkohol, 2-Chlorethanol, 2,2,2-Trichlorethanol, Cyclohexanol, Benzylalkohol, Propylalkohol, tert.-Butylalkohol, Cyclopropylmethylalkohol, 3-Butin-2-ol, 2-Methyl-3-butin-2-ol, 2-Methyl-3-butin-2-ol, Ethylmercaptan.

Als Lösungsmittel kommen für die Erfindungsgemäße Umsetzung gemäß Verfahrensvariante (a) und (b) vorzugsweise inerte organische Lösungsmittel infrage. Hierzu gehören vorzusweise Ketone, wie Diethylketon, insbesondere Aceton und Methylisobutylketon: Nitrile, wie Propionitril, insbesondere Acetonitril; Ether, wie Tetrahydrofuran oder Dioxan; aliphatische und aromatische Kohlenwasserstoffe, wie Petrolether, Benzol, Toluol oder Xylol, halogenierte Kohlenwasserstoffe, wie Methylenchlorid, Tetrachlorkohlenstoff, Chloroform oder Chlorbenzol; und Ester, wie Essigester.

Die erfindungsgemäßen Verfahren (a) und (b) können gegebenenfalls in Gegenwart von Säurebindern (Chlorwasserstoff-Akzeptoren) durchgeführt werden; als solche können alle üblichen Säurebindungsmittel verwendet werden. Hierzu gehören organische Basen, vorzugsweise tertiäre Amine, wie z.B. Triäthylamin; ferner anorganische Basen, wie z.B. Alkalihydroxide und Alkalicarbonate. Als Katalysator kommt insbesondere Dimethylformamid in Betracht.

Die Reaktionstemperaturen können bei der Durchführung der erfindungsgemäßen Verfahren (a)

und (b) in einem größeren Bereich variiert werden. Im allgemeinen arbeitet man zwischen 0 und 120°C, vorzugsweise zwischen 20 und 100°C.

Bei der Durchführung des erfindungsgemäßen Verfahrens (a) setzt man vorzugsweise auf 1 Mol der Verbindungen der Formel (II) 1 bis 1,5 Mol Chlorglyoxylsäureester der Formel (III) und gegebenenfalls 1 bis 1,5 Mol Säurebinder ein. Die Isolierung der Verbindungen der Formel (I) erfolgt in üblicher Weise.

Bei der Durchführung des erfindungsgemäßen Verfahrens (b) setzt man vorzugsweise auf 1 Mol der Verbindungen der Formel (II) 2 Mol Oxalylchlorid der Formel (IV) und ca. 1,1 Mol Alkohol bzw. Mercaptan der Formel (VI) ein. Die Isolierung der Verbindungen der Formel (I) erfolgt in üblicher Weise.

Nach einer besonderen Ausführungsform können Verbindungen der Formel (I), in der $R^6$ für die Gruppierung

$$[-X-\overset{\oplus}{N}R^7R^8R^9]\ Z^{\ominus}$$

steht, erhalten werden, indem man entsprechende erfindungsgemäße Verbindungen, in denen $R^6$ für ein tertiäres Amin steht, mit Alkylhalogeniden in Gegenwart eines polaren organischen Lösungsmittels, wie beispielsweise Acetonitril oder Nitromethan, bei Temperaturen zwischen 20 und 120°C, vorzugsweise bei der Siedetemperatur des jeweiligen Lösungsmittels, umsetzt und gegebenenfalls das Halogenid in den erhaltenen Salzen in an sich bekannter Weise gegen ein anderes Anion austrauscht, indem man die Halogenide der Formel (I) z.B. mittels einer Base oder einem Anionenaustauscherharz in die entsprechenden Hydroxide überführt und diese anschließend mit einer entsprechenden Säure umsetzt.

In einer weiteren besonderen Ausführungsform können Verbindungen der Formel (I), in der $R^6$ für die Gruppierung

$$-X-\overset{\overset{O}{\uparrow}}{N}R^7R^8$$

steht, erhalten werden, indem man entsphrechende erfindungsgemäße Verbindungen, in denen $R^6$ für ein tertiäres Amin steht, in an sich bekannter Weise mit Peroxiden, wie beispielsweise Wasserstoffperoxid, tert.-Butylhydroperoxid oder 3-Chlorperbenzosäure, gegebenenfalls in Gegenwart eines Katalysators, wie z.B. Vanadin-, Molybdän- oder Titanverbindungen, und gegebenenfalls in Gegenwart eines organischen Lösungsmittels, wie beispielsweise Eisessig oder Methylenchlorid, bei Temperaturen zwischen 20 und 80°C zu den N-Oxiden umsetzt.

In einer weiteren besonderen Ausführungsform können Verbindungen der Formel (I), in der $R^6$ für Epoxialkyl steht, erhalten werden, indem entsprechende erfindungsgemäße Verbindungen, in denen $R^6$ für Alkenyl steht, in an sich bekannter Weise durch Einwirkung von Persäuren, wie beispielsweise Peressigsäure, Perbenzoesäure oder Trifluorperessigsäure, epoxidiert werden.

Die erfindungsgemäßen Wirkstoffe weisen eine starke mikrobizide Wirkung auf und können zur Bekämpfung von unerwünschten Mikroorganismen praktisch eigesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basiodiomycetes, Deuteromycetes.

Die gute Pflanzenverträglichkeit der Wirkstoffe in den zur Bekämpfung von Pflanzenkrankheiten notwendigen Konzentationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung falscher Mehltaupilze, z.B. des Erregers der Kraut- und Braunfäule der Tomate und Kartoffel (Phytophthora infestans) eingesetzt werden. Besonders hervorzuheben ist, daß die erfindungsgemäßen Wirkstoffe nicht nur eine protektive Wirkung entfalten, sondern auch systemisch wirksam sind. So gelingt es, Pflanzen gegen Pilzbefall zu schützen, wenn man den Wirkstoff über den Boden und die Wurzel oder über das Saatgut den oberirdischen Teilen der Pflanze zuführt.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Tuluol, oder Alkylnaphthaline, chlorierte Aromaten oder chlorierte aliphatische Kohlenwasserstoffe, wie

Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hoch-disperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10, g, benötigt.

Bei der Behandlung des Bodens sind Wirkstoffkonzentrationen von 0,00001 bis 0,1 Gew.-%, vorzugsweise von 0,0001 bis 0,02%, am Wirkungsort erforderlich.

Mit nachfolgenden Beispielen werden einige Verwendungsmöglichkeiten eingehender dargestellt:

## Beispiel A

Phytophthora-Test (Tomaten)/Protektiv

Lösungsmittel: 4,7 Gew.-Teile Aceton
Emulgator: 0,3 Gew.-Teile Alkylarylpolyglykolether
Wasser: 95,0 Gew.-Teile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Tomaten-pflanzen mit 2 bis 4 Laubblättern bis zur Tropfnässe. Die Pflanzen verbleiben 24 Stunden bei 20°C und einer relativen Luftfeuchtigkeit von 70% Im Gewächshaus. Anschließend werden die Tomatenpflanzen mit einer wäßrigen Sporensuspension von Phytophthora infestans inokuliert. Die Pflanzen werden in eine Feuchtkammer mit einer 100%igen Luftfeuchtigkeit und einer Temperatur von 18 bis 20°C gebracht.

Nach 5 Tagen wird der Befall der Tomatenpflanzen bestimmt. Die erhaltenen Boniturwerte werden auf Prozent Befall umgerechnet. 0% bedeutet keinen Befall, 100% bedeutet, daß die Pflanzen vollständig befallen sind.

Wirkstoff, Wirkstoffkonzentrationen und Ergebnisse werden ermittelt.

In diesem Test zeigen z.B. folgende Verbindungen eine sehr gute Wirkung, die derjenigen der aus dem Stand der Technik bekannten Verbindungen deutlich überlegen ist:

Verbindungen gemäß Herstellungsbeispielen 1, 3, 4, 5, 6, 7, 11, 12, 15, 17, 18 und 34.

Beispiel B

Phytophthora-Rest (Tomaten)/Systemisch

Lösungsmittel: 4,7 Gew.-Teile Aceton
Dispergiermittel: 0,3 Gew.-Teile Alkylarylpolyglykolether
Wasser: 95,0 Gew.-Teile

Man vermischt die für die gewünschte Wirkstoffkonzentration in der Gießflüssigkeit nötige Wirkstoffmenge mit der angegebenen Menge des Lösungsmittels und verdünnt das Konzentrat mit der angegebenen Menge Wasser, welches die genannten Zusätze enthält.

In Einheitserde angezogene Tomatenpflanzen mit 2 bis 4 Laubblättern werden innerhalb einer Woche dreimal mit 10 ccm der Gießflüssigkeit in der angegebenen Wirkstoffkonzentration, bezogen auf 100 ccm Erde, gegossen.

Die so behandelten Pflanzen werden nach der Behandlung mit einer wäßrigen Sporensuspension von Phytophthora infestans inokuliert. Die Pflanzen werden in eine Feuchtekammer mit einer Luftfeuchtigkeit von 100% und einer Temperatur von 18 bis 20°C gebracht. Nach 5 Tagen wird der Befall der Tomatenpflanzen bestimmt. Die erhaltenen Boniturwerte werden auf Prozent Befall umgerechnet. 0% bedeutet keinen Befall, 100% bedeutet, daß die Pflanzen vollständig befallen sind.

Wirkstoffe, Wirkstoffkonzentrationen und Ergebnisse werden ermittelt. Bei diesem Test zeigen z.B. folgende Verbindungen eine sehr gute Wirkung, die derjenigen der aus dem Stand der Technik bekannten Verbindungen überlegen ist: Verbindungen gemäß Herstellungsbeispielen 1 und 34.

Herstellungsbeispiele

Beispiel 1

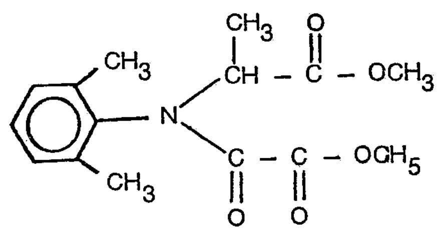

(Verfahren a)

Zu einer Lösung von 20,7 g (0,1 Mol) N-(2,6-Xylyl)-alaninmethylester in 100 ml Toluol gibt man einige Tropfen Dimethylformamid, tropft in 15 Minuten bei 20—30°C 18,4 g (0,15 Mol) Methoxalylchlorid zu und rührt 1,5 Stunden bei Raumtemperatur nach. Die Reaktionslösung wird mit Essigester verdünnt, mit 20%-iger Salzsäure, Wasser und 10%-iger Natriumhydrogencarbonatlösung gewaschen, über Natriumsulfat getrocknet und eingedampft. Den Rückstand kristallisiert man aus Ligroin um. Man erhält so 20,8 g (71% der Theorie) N-Methoxalyl-N-(2,6-xylyl)-alaninmethylester vom Schmelzpunkt 69—70°C.

Beispiel 2

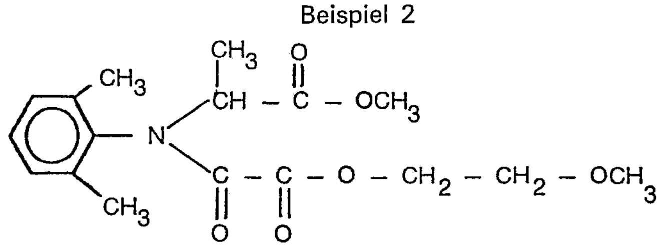

(Verfahren b)

In eine Lösung von 12,7 g (0,1 Mol) Oxalylchlorid in 50 ml Toluol tropft man in 10 Minuten bei 20—25°C eine Lösung von 10,5 g (0,05 Mol) N-(2,6-Xylyl)-alaninmethylester und einigen Tropfen Dimethylformamid in 25 ml Toluol und rührt 30 Minuten bei Raumtemperatur nach. Es wird eingedampft und zur Entfernung von Oxalylchlorid nochmals mit Toluol abgedampft.

Den Rückstand löst man in 50 ml Toluol, tropft in 7 Minuten bei 25°C 4,6 g (0,06 Mol) Methylglykol zu und rührt 3 Stunden bei 50°C. Die Reaktionslösung wird mit Essigester verdünnt, mit 10%igem wässrigem Pyridin, mit 20%iger Salzsäure, mit Wasser und mit 10%iger Hydrogencarbonat-Lösung gewaschen, über Natriumsulfat getrocknet und eingedampft. Den Rückstand kristallisiert man aus Toluol/Ligroin (1:15) um. Man erhält so 10,7 g (64% der Theorie) N-(2-Methoxyethoxalyl)-N-(2,6-Xylyl)-alaninmethylester vom Schmelzpunkt 53—54°C.

Analog werden die nachfolgenden Verbindungen der allgemeinen Formel

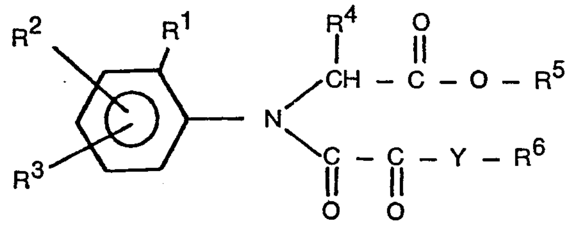

(I)

erhalten:

TABELLE 1 (Fortsetzung)

| Bsp. Nr. | $R^1$ | $R^2$ | $R^3$ | $R^4$ | $R^5$ | $R^6$ | Y | Fp (°C) bzw. Brechungsindex |
|---|---|---|---|---|---|---|---|---|
| 3 | i-$C_3H_7$ | H | 6-i-$C_3H_7$ | $CH_3$ | $-CH_2-CH_2-OCH_3$ | $CH_3$ | O | $n_D^{20}$ : 1,5011 |
| 4 | $C_2H_5$ | H | 6-$C_2H_5$ | $CH_3$ | $-CH_2-CH_2-OCH_3$ | $CH_3$ | O | $n_D^{20}$ : 1,5038 |
| 5 | $CH_3$ | H | 6-$CH_3$ | $CH_3$ | i-$C_3H_7$ | $CH_3$ | O | $n_D^{20}$ : 1,4966 |
| 6 | $CH_3$ | H | 6-$C_2H_5$ | $CH_3$ | $-CH_2-CH_2-OCH_3$ | $CH_3$ | O | $n_D^{20}$ : 1,5028 |
| 7 | $C_2H_5$ | 4-$CH_3$ | 6-$C_2H_5$ | $CH_3$ | $-CH_2-CH_2-OCH_3$ | $CH_3$ | O | 83–84 |
| 8 | $CH_3$ | 4-$CH_3$ | 6-$CH_3$ | $CH_3$ | $-CH_2-CH_2-OCH_3$ | $CH_3$ | O | 78–79 |
| 9 | $CH_3$ | 4-$CH_3$ | 6-$C_2H_5$ | $CH_3$ | $C_2H_5$ | $CH_3$ | O | 92–93 |
| 10 | $CH_3$ | 4-$CH_3$ | 6-$C_2H_5$ | $CH_3$ | $-CH_2-CH_2-OCH_3$ | $CH_3$ | O | 66–67 |
| 11 | $CH_3$ | 4-$CH_3$ | 6-$CH_3$ | $CH_3$ | $CH_3$ | $CH_3$ | O | $n_D^{20}$: 1,5086 |
| 12 | $CH_3$ | 4-$CH_3$ | 6-$C_1H_5$ | $CH_3$ | $CH_3$ | $CH_3$ | O | $n_D^{19}$ : 1,5098 |
| 13 | $CH_3$ | H | 5-$CH_3$ | $CH_3$ | $CH_3$ | $CH_3$ | O | $n_D^{20}$ : 1,5060 |
| 14 | H | 4-$CH_3$ | H | $CH_3$ | $CH_3$ | $CH_3$ | O | $n_D^{20}$ : 1,5086 |
| 15 | $CH_3$ | 3-$CH_3$ | 5-$CH_3$ | $CH_3$ | $CH_3$ | $CH_3$ | O | $n_D^{20}$ : 1,5026 |
| 16 | $CH_3$ | H | 6-$CH_3$ | 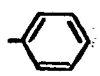 | $CH_3$ | $CH_3$ | O | 115–17 |
| 17 | i-$C_3H_7$ | H | H | $CH_3$ | $CH_3$ | $CH_3$ | O | $n_D^{20}$ : 1,5060 |
| 18 | sek.-$C_4H_9$ | H | H | $CH_3$ | $CH_3$ | $CH_3$ | O | $n_D^{20}$ · 1,5018 |
| 19 | $CH_3$ | H | H | $CH_3$ | $CH_3$ | $CH_3$ | O | $n_D^{20}$ : 1,5071 |
| 20 | $C_2H_5$ | H | H | $CH_3$ | $CH_3$ | $CH_3$ | O | $n_D^{20}$ : 1,5071 |
| 21 | $CH_3$ | 3-$CH_3$ | H | $CH_3$ | $CH_3$ | $CH_3$ | O | $n_D^{20}$ : 1,5094 |
| 22 | $CH_3$ | H | 6-$CH_3$ | $CH_3$ | $CH_3$ | $C_2H_5$ | O | 89–90 |
| 23 | $CH_3$ | H | 6-$CH_3$ | $CH_3$ | $CH_3$ | 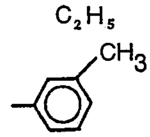 | O | $n_D^{19}$ : 1,5323 |
| 24 | $CH_3$ | H | 6-$CH_3$ | $CH_3$ | $CH_3$ | 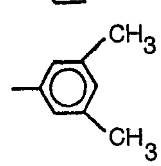 | O | 96–99 |
| 25 | $CH_3$ | H | 6-$CH_3$ | $CH_3$ | $CH_3$ | 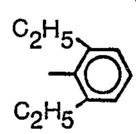 | O | 72–74 |
| 26 | $CH_3$ | H | 6-$CH_3$ | $CH_3$ | $CH_3$ | i-$C_3H_7$ | O | 68–72 |

TABELLE 1 (Fortsetzung)

| Bsp. Nr. | $R^1$ | $R^2$ | $R^3$ | $R^4$ | $R^5$ | $R^6$ | Y | Fp (°C) bzw. Brechungsindex |
|---|---|---|---|---|---|---|---|---|
| 27 | $CH_3$ | H | 6-$CH_3$ | $CH_3$ | $CH_3$ | 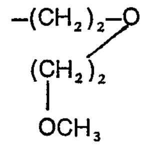 | O | $n_D^{21}$ : 1,5038 |
| 28 | $CH_3$ | H | 6-$CH_3$ | $CH_3$ | $CH_3$ | 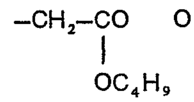 | O | $n_D^{21}$ : 1,4947 |
| 29 | $CH_3$ | H | 6-$CH_3$ | $CH_3$ | $CH_3$ | $-CH_2-CH_2CN$ | O | 85–87 |
| 30 | $CH_3$ | H | 6-$CH_3$ | $CH_3$ | $CH_3$ | 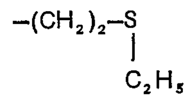 | O | $n_D^{22}$ : 1,5203 |
| 31 | $CH_3$ | H | 6-$CH_3$ | $CH_3$ | $CH_3$ | 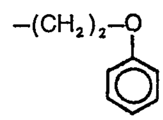 | O | $n_D^{22}$ : 1,5341 |
| 32 | $CH_3$ | H | 6-$CH_3$ | $CH_3$ | $CH_3$ | 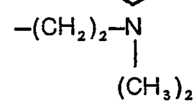 | O | 108–12 (xHCl) |
| 33 | $CH_3$ | H | 6-$CH_3$ | $CH_3$ | $CH_3$ | 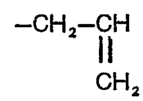 | O | 77–79 |
| 34 | $CH_3$ | H | 6-$CH_3$ | $CH_3$ | $CH_3$ | $-CH_2-CH\equiv CH$ | O | $n_D^{24}$ : 1,5164 |
| 35 | $CH_3$ | H | 6-$CH_3$ | $CH_3$ | $CH_3$ | $-CH_2-CH_2Cl$ | O | 79–80 |
| 36 | $CH_3$ | H | 6-$CH_3$ | $CH_3$ | $CH_3$ | 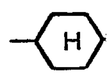 | O | $n_D^{22}$ : 1,5123 |
| 37 | $CH_3$ | H | 6-$CH_3$ | $CH_3$ | $CH_3$ | 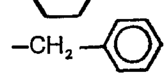 | O | $n_D^{22}$ : 1,5373 |
| 38 | $CH_3$ | H | 6-$CH_3$ | $CH_3$ | $CH_3$ | $-C_4H_9$ | S | $n_D^{25}$ : 1,5270 |
| 39 | $CH_3$ | H | 6-$CH_3$ | $CH_3$ | $CH_3$ | $-CH_2-CCl_3$ | O | 64–65 |
| 40 | $CH_3$ | H | 6-$CH_3$ | $CH_3$ | $CH_3$ | $C_3H_7$ | O | 83–84 |
| 41 | $CH_3$ | H | 6-$CH_3$ | $CH_3$ | $CH_3$ | $-CH(CH_3)-C\equiv CH$ | O | 01 |
| 42 | $CH_3$ | H | 6-$CH_3$ | $CH_3$ | $CH_3$ | 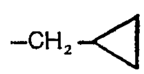 | O | 53–54 |
| 43 | $CH_3$ | H | 6-$CH_3$ | $C_2H_5$ | $CH_3$ | $CH_3$ | O | 105–06 |

**Patentansprüche**

1. N-Oxalyl-Derivate von N-Phenyl-aminosäure(n) (estern) der allgemeinen Formel

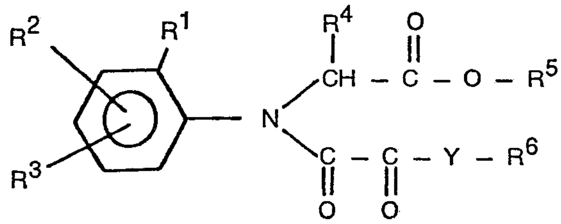

(I)

in welcher

$R^1$ für Wasserstoff, Alkyl mit 1 bis 4 C-Atomen oder Halogen steht,

$R^2$ für Wasserstoff oder Alkyl mit 1 bis 4 C-Atomen steht,

$R^3$ für Wasserstoff oder Alkyl mit 1 bis 4 C-Atomen steht,

$R^4$ für Wasserstoff, Alkyl mit 1 bis 4 C-Atomen oder gegebenenfalls substituiertes Phenyl steht,

$R^5$ für Wasserstoff, Alkyl, und Cyanalkyl, mit 1 bis 6 C-Atomen, Alkenyl, und Alkinyl, mit 2 bis 4 C-Atomen, Halogenalkyl, mit 1 bis 4 C-Atomen, Cycloalkyl und Cycloalkylalkyl, mit 3 bis 7 C-Atomen im Cycloalkylteil und 1 bis 4 C-Atomen im Alkylteil, Alkoxyalkyl, Alkoxyalkoxyalkyl, Alkoxycarbonylalkyl, Alkylthioalkyl, Alkylsulfinylalkyl und Alkylsulfonylalkyl mit jeweils 1 bis 4 C-Atomen in jedem Alkylteil, Dialkylaminoalkyl mit 1 bis 4 C-Atomen in jedem Alkylteil oder gegebenenfalls substituiertes Aryl mit 6 bis 10 C-Atomen und Aralkyl mit 6 bis 10 C-Atomen im Arylteil und 1 bis 4 C-Atomen im Alkylteil steht,

$R^6$ für Alkyl und Cyanalkyl mit 1 bis 6 C-Atomen, Alkenyl und Alkinyl mit 2 bis 4 C-Atomen, Halogenalkyl mit 1 bis 4 C-Atomen, Cycloalkyl und Cycloalkylalkyl mit 3 bis 7 C-Atomen im Cycloalkylteil und 1 bis 4 C-Atomen im Alkylteil, Alkoxyalkyl, Alkoxyalkoxyalkyl, Alkoxycarbonylalkyl, Alkylthioalkyl, Alkylsulfinylalkyl und Alkylsulfonylalkyl mit jeweils 1 bis 4 C-Atomen in jedem Alkylteil, Dialkylaminoalkyl mit 1 bis 4 C-Atomen in jedem Alkylteil, gegebenenfalls substituiertes Aryl mit 6 bis 10 C-Atomen, Aralkyl oder Aroxyalkyl mit 6 bis 10 C-Atomen im Arylteil und 1 bis 4 C-Atomen im Alkylteil, Epoxialkyl mit 3 bis 8 C-Atomen sowie die Gruppierungen

$$[—X—\overset{\oplus}{N}R^7R^8R^9]\ Z^{\ominus} \quad \text{und} \quad —X—\overset{\overset{O}{\uparrow}}{N}R^7R^8$$

steht, wobei

$R^7$, $R^8$ und $R^9$ gleich oder verschieden sind und für Alkyl mit 1 bis 4 C-Atomen stehen,

X für Alkylen mit 1 bis 4 C-Atomen oder Alkyliden mit 2 bis 4 C-Atomen steht,

Y für Sauerstoff oder Schwefel steht und

Z für das Anion einer anorganischen oder organischen Säure steht,

2. N-Oxalyl-Derivate von N-Phenyl-aminosäure(n) (estern) der allgemeinen Formel

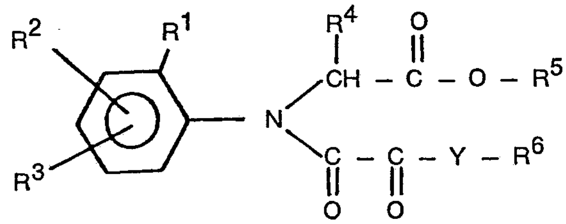

(I)

in welcher

$R^1$ für Wasserstoff, Alkyl mit 1 bis 4 C-Atomen oder Halogen steht,

$R^2$ für Wasserstoff oder Alkyl mit 1 bis 4 C-Atomen steht,

$R^3$ für Wasserstoff oder Alkyl mit 1 bis 4 C-Atomen steht,

$R^4$ für Wasserstoff, Alkyl mit 1 bis 4 C-Atomen oder gegebenenfalls mit Halogen, Alkyl mit 1 bis 2 C-Atomen und Halogenalkyl mit 1 bis 2 C-Atomen substituiertes Phenyl steht,

$R^5$ für Wasserstoff, Alkyl, und Cyanalkyl mit 1 bis 6 C-Atomen, Alkenyl und Alkinyl mit 2 bis 4 C-Atomen, Halogenalkyl mit 1 bis 4 C-Atomen, Cycloalkyl und Cycloalkylalkyl mit 3 bis 7 C-Atomen im Cycloalkylteil und 1 bis C-Atomen im Alkylteil, Alkoxyalkyl, Alkoxyalkoxyalkyl, Alkoxycarbonylalkyl, Alkylthioalkyl, Alkylsulfinylalkyl und Alkylsulfonylalkyl mit jeweils 1 bis 4 C-Atomen in jedem Alkylteil, Diaalkylaminoalkyl mit 1 bis 4 C-Atomen in jedem Alkylteil oder gegebenenfalls mit Halogen, Alkyl mit 1 bis 2 C-Atomen und Halogenalkyl mit 1 bis 2 C-Atomen substituiertes Aryl mit 6 bis 10 C-Atomen und Aralkyl mit 6 bis 10 C-Atomen im Arylteil und 1 bis 4 C-Atomen im Alkylteil steht,

$R^6$ für Alkyl und Cyanalkyl, mit 1 bis 6 C-Atomen, Alkenyl und Alkinyl mit 2 bis 4 C-Atomen, Halogenalkyl mit 1 bis 4 C-Atomen, Cycloalkyl und Cycloalkylalkyl mit 3 bis 7 C-Atomen im Cycloalkylteil und

1 bis 4 C-Atomen im Alkylteil, Alkoxyalkyl, Alkoxyalkoxyalkyl, Alkoxycarbonylalkyl, Alkylthioalkyl, Alkylsulfinylalkyl und Alkylsulfonylalkyl mit jeweils 1 bis 4 C-Atomen in jedem Alkylteil, Dialkylaminoalkyl mit 1 bis 4 C-Atomen in jedem Alkylteil, gegebenenfalls mit Halogen, Alkyl mit 1 bis 2 C-Atomen und Halogenalkyl mit 1 bis 2 C-Atomen substituiertes Aryl mit 6 bis 10 C-Atomen, Aralkyl oder Aroxyalkyl mit 6 bis 10 C-Atomen im Arylteil und 1 bis 4 C-Atomen im Alkylteil, Epoxialkyl mit 3 bis 8 C-Atomen sowie die Gruppierungen

$$[\text{—X—}\overset{\oplus}{N}R^7R^8R^9]\ Z^{\ominus} \quad \text{und} \quad \text{—X—}\overset{\overset{O}{\uparrow}}{N}R^7R^8$$

steht, wobei

$R^7$, $R^8$ und $R^9$ gleich oder verschieden sind und für Alkyl mit 1 bis 4 C-Atomen stehen,

X für Alkylen mit 1 bis 4 C-Atomen oder Alkyliden mit 2 bis 4 C-Atomen steht,

Y für Sauerstoff oder Schwefel steht und

Z für das Anion einer anorganischen oder organischen Säure steht,

3. Verfahren zur Herstellung von N-Oxalyl-Derivaten von N-Phenyl-aminosäure(n) (estern) der allgemeinen Formel

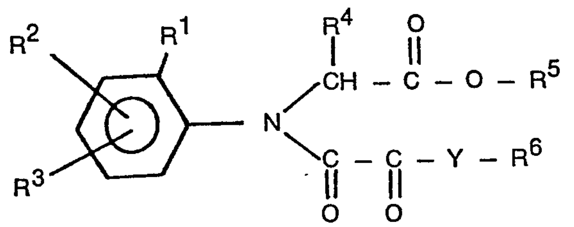

(I)

in welcher

$R^1$ für Wasserstoff, Alkyl mit 1 bis 4 C-Atomen oder Halogen steht,

$R^2$ für Wasserstoff oder Alkyl mit 1 bis 4 C-Atomen steht,

$R^3$ für Wasserstoff oder Alkyl mit 1 bis 4 C-Atomen steht,

$R^4$ für Wasserstoff, Alkyl mit 1 bis 4 C-Atomen oder gegebenenfalls substituiertes Phenyl steht,

$R^5$ für Wasserstoff, Alkyl und Cyanalkyl mit 1 bis 6 C-Atomen, Alkenyl und Alkinyl mit 2 bis 4 C-Atomen, Halogenalkyl mit 2 bis 4 C-Atomen, Cycloalkyl und Cycloalkylalkyl mit 3 bis 7 C-Atomen im Cycloalkylteil und 1 bis 4 C-Atomen im Alkylteil, Alkoxyalkyl, Alkoxyalkoxyalkyl, Alkoxycarbonylalkyl, Alkylthioalkyl, Alkylsulfinylalkyl und Alkylsulfonylalkyl mit jeweils 1 bis 4 C-Atomen in jedem Alkylteil, Dialkylaminoalkyl mit 1 bis 4 C-Atomen in jedem Alkylteil oder gegebenenfalls substituiertes Aryl mit 6 bis 10 C-Atomen und Aralkyl mit 6 bis 10 C-Atomen im Arylteil und 1 bis 4 C-Atomen im Alkylteil steht,

$R^6$ für Alkyl und Cyanalkyl mit 1 bis 6 C-Atomen, Alkenyl, Alkinyl und Alkinyl mit 2 bis 4 C-Atomen, Halogenalkyl mit 1 bis 4 C-Atomen, Cycloalkyl und Cycloalkylalkyl mit 3 bis 7 C-Atomen im Cycloalkylteil und 1 bis 4 C-Atomen im Alkylteil, Alkoxyalkyl, Alkoxyalkoxyalkyl, Alkoxycarbonylalkyl, Alkylthioalkyl, Alkylsulfinylalkyl und Alkylsulfonylalkyl mit jeweils 1 bis 4 C-atomen in jedem Alkylteil, Dialkylaminoalkyl mit 1 bis 4 C-Atomen in jedem Alkylteil, gegebenenfalls mit Halogen, Alkyl mit 1 bis 2 C-Atomen und Halogenalkyl mit 1 bis 2 C-Atomen substituiertes Aryl mit 6 bis 10 C-Atomen, Aralkyl oder Aroxyalkyl mit 6 bis 10 C-Atomen im Arylteil und 1 bis 4 C-Atomen im Alkylteil, Epoxialkyl mit 3 bis 8 C-Atomen sowie die Gruppierungen

$$[\text{—X—}\overset{\oplus}{N}R^7R^8R^9]\ Z^{\ominus} \quad \text{und} \quad \text{—X—}\overset{\overset{O}{\uparrow}}{N}R^7R^8$$

steht, wobei

$R^7$, $R^8$ und $R^9$ gleich oder verschieden sind und für Alkyl mit 1 bis 4 C-Atomen stehen,

X für Alkylen mit 1 bis 4 C-Atomen oder Alkyliden mit 2 bis 4 C-Atomen steht,

Y für Sauerstoff oder Schwefel steht und

Z für das Anion einer anorganischen oder organischen Säure steht,

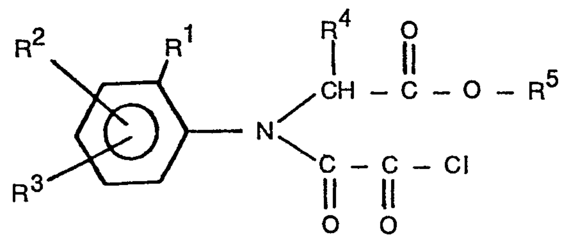

(V)

in welcher

$R^1$ bis $R^5$ die in Anspruch 1 angegebene Bedeutung haben, mit Alkoholen bzw. Mercaptanen der Formel

$$H—Y—R^6 \quad (VI)$$

in welcher

$R^6$ und Y die in Anspruch 1 angegebene Bedeutung haben, in Gegenwart eines Lösungsmittels und gegebenenfalls in Gegenwart eines Säurebindemittels und/oder in Gegenwart eines Katalysators umsetzt dadurch gekennzeichnet, daß man N-Phenylaminosäure(n) (ester) der Formel

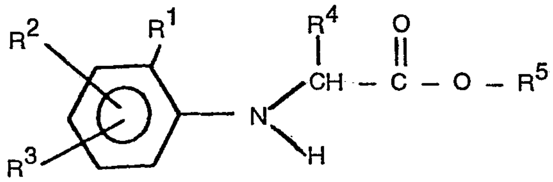

in welcher

$R^1$ bis $R^5$ die obenangegebene Bedeutung haben,

a) mit Chlorglyoxylsäureestern der Formel

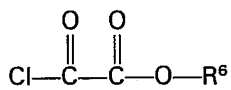

(III)

in welcher

$R^6$ die obenangegebene Bedeutung hat, in Gegenwart eines Lösungsmittels und gegebenenfalls in Gegenwart eines Säurebindemittels und/oder in Gegenwart eines Katalysators umsetzt, oder

b) zunächst mit Oxalylchlorid der Formel

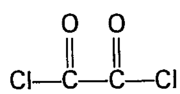

(IV)

in Gegenwart eines Lösungsmittels und gegebenenfalls in Gegenwart eines Säurebindemittels und/oder in Gegenwart eines Katalysators umsetzt und anschließend die dabei entstehenden Verbindungen der Formel.

4. Verfahren zur Herstellung von N-Oxalkyl-Derivaten von N-Phenyl-aminosäure(n) (Estern) der allgemeinen Formel

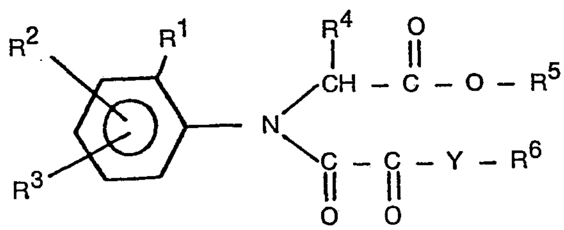

(I)

in welcher

$R^1$ für Wasserstoff, Alkyl mit 1 bis 4 C-Atomen oder Halogen steht,

$R^2$ für Wasserstoff oder Alkyl mit 1 bis 4 C-Atomen steht,

$R^3$ für Wasserstoff oder Alkyl mit 1 bis 4 C-Atomen steht,

$R^4$ für Wasserstoff, Alkyl mit 1 bis 4 C-Atomen oder gegebenenfalls mit Halogen, Alkyl mit 1 bis 2 C-Atomen und Halogenalkyl mit 1 bis 2 C-Atomen substituiertes Phenyl steht,

$R^5$ für Wasserstoff, Alkyl und Cyanalkyl mit 1 bis 6 C-Atomen, Alkenyl und Alkinyl mit 2 bis 4 C-Atomen, Halogenalkyl mit 1 bis 4 C-Atomen, Cycloalkyl und Cycloalkylalkyl mit 3 bis 7 C-Atomen im Cycloalkylteil und 1 bis 4 C-Atomen im Alkylteil, Alkoxyalkyl, Alkoxyalkoxyalkyl, Alkoxycarbonylalkyl, Alkylthioalkyl, Alkylsulfinylalkyl und Alkylsulfonylalkyl mit jeweils 1 bis 4 C-Atomen in jedem Alkylteil, Dialkylaminoalkyl mit 1 bis 4 C-Atomen in jedem alkylteil oder gegebenenfalls mit Halogen, Alkyl mit 1 bis 2 C-Atomen und Halogenalkyl mit 1 bis 2 C-Atomen und Halogenalkyl mit 1 bis 2 C-Atomen substituiertes Aryl mit 6 bis 10 C-Atomen und Aralkyl dadurch gekennzeichnet, daß mit man N-Phenylaminosäure(n) (ester) der Formel

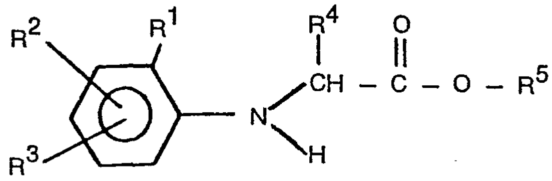

in welcher

$R^1$ bis $R^5$ die obenangegebene Bedeutung haben,

a) mit Chlorglyoxylsäureestern der Formel

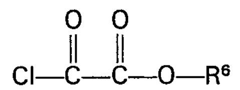

(III)

in welcher

$R^6$ die oben angegebene Bedeutung hat, in Gegenwart eines Lösungsmittels und gegebenenfalls in Gegenwart eines Säurebindemittels und/oder in Gegenwart eines Katalysators umsetzt, oder

b) zunächst mit Oxalylchlorid der Formel

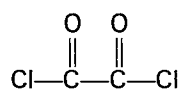

(IV)

in Gegenwart eines Lösungsmittels und gegebenenfalls in Gegenwart eines Säurebindemittels und/oder in Gegenwart eines Katalysators umsetzt und anschließend die dabei entstehenden Verbindungen der Formel

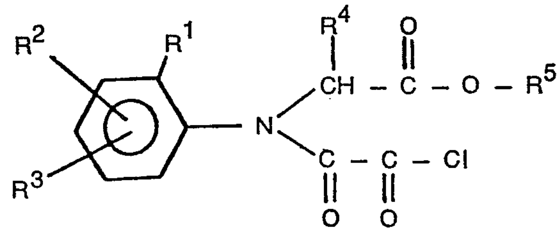

(V)

in welcher

$R^1$ bis $R^5$ die obenangegebene Bedeutung haben,

mit Alkoholen bzw. Mercaptanen der Formel

$$H—Y—R^6 \qquad (VI)$$

in welcher

$R^6$ und Y die oben angegebene Bedeutung haben,

in Gegenwart eines Lösungsmittels und gegebenenfalls in Gegenwart eines Säurebindemittels und/oder in Gegenwart eines Katalysators umsetzt.

5. Fungizide Mittel, gekennzeichnet durch einen Gehalt an mindestens einem N-Oxalyl-Derivat von N-Phenylaminosäure(n) (estern) gemaß Anspruch 1.

6. Verfahren zur Bekämpfung von Pilzen, dadurch gekennzeichnet, daß man N-Oxalyl-Derivate von N-Phenylaminosäure(n) (estern) gemäß Anspruch 1 und 2 auf Pilze oder ihren Lebensraum einwirken läßt.

7. Verwendung von N-Oxalyl-Derivaten von N-Phenyl-aminosäure(n) (estern) gemäß Anspruch 1 und 2 zur Bekämpfung von Pilzen.

8. Verfahren zur Herstellung von fungiziden Mitteln, dadurch gekennzeichnet, daß man N-Oxalyl-Derivate von N-Phenyl-aminosäure(n) (estern) gemäß Anspruch 1 und 2 mit Streckmitteln und/oder oberflächenaktiven mitteln vermischt.

**Revendications**

1. Dérivés N-oxalyle de N-phényl-aminoacides(esters) de formule générale:

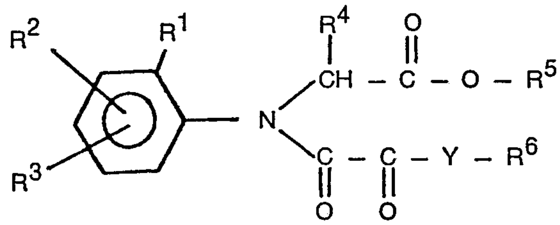

(I)

dans laquelle

$R^1$ représente un atome d'hydrogène, un groupe alkyle contenant 1 à 4 atomes de carbone, ou un atome d'halogène,

$R^2$ représente un atome d'hydrogène ou un groupe alkyle contenant 1 à 4 atomes de carbone,

$R^3$ représente un atome d'hydrogène ou un groupe alkyle contenant 1 à 4 atomes de carbone,

$R^4$ représente un atome d'hydrogène, un groupe alkyle contenant 1 à 4 atomes de carbone ou un groupe phényle éntuellement substitué,

$R^5$ représente un atome d'hydrogène, un groupe alkyle ou un groupe cyanalkyle contenant 1 à 6 atomes de carbone, un groupe alcényle ou un groupe alcinyle contenant 2 à 4 atomes de carbone, un groupe halogénalkyle contenant 1 à 4 atomes de carbone, un groupe cycloalkyle ou un groupe cycloalkylalkyle contenant 3 à 7 atomes de carbone dans la fraction cycloalkyle et 1 à 4 atomes de carbone dans la fraction alkyle, un groupe alcoxyalkyle, un groupe alcoxyalcoxyalkyle, un groupe alcoxycarbonylalkyle, un groupe alkylthioalkyle, un groupe alkylsulfinylalkyle ou un groupe alkylsulfonylalkyle contenant chaque fois 1 à 4 atomes de carbone dans chaque fraction alkyle, un groupe dialkylaminoalkyle contenant 1 à 4 atomes de carbone dans chaque fraction alkyle ou un groupe aryle éventuellement substitué et contenant 6 à 10 atomes de carbone, ainsi qu'un groupe aralkyle contenant 6 à 10 atomes de carbone dans la fraction aryle et 1 à 4 atomes de carbone dans la fraction alkyle,

$R^6$ représente un groupe alkyle ou un groupe cyanalkyle contenant 1 à 6 atomes de carbone, un groupe alcényle ou un groupe alcinyle contenant 2 à 4 atomes de carbone, un groupe halogènalkyle contenant 1 à 4 atomes de carbone, un groupe cycloalkyle ou un groupe cycloalkylalkyle contenant 3 à 7 atomes de carbone dans la fraction cycloalkyle et 1 à 4 atomes de carbone dans la fraction alkyle, un groupe alcoxyalkyle, un groupe alcoxyalcoxyalkyle, un groupe alcoxycarbonylalkyle, un groupe alkylthioalkyle, un groupe alkylsulfinylalkyle ou un groupe alkylsulfonylalkyle contenant chaque fois 1 à 4 atomes de carbone dans chaque fraction alkyle, un groupe dialkylaminoalkyle contenant 1 à 4 atomes de carbone dans chaque fraction alkyle, un groupe aryle éventuellement substitué et contenant 6 à 10 atomes de carbone, un groupe aralkyle ou un groupe aroxyalkyle contenant 6 à 10 atomes de carbone dans la fraction aryle et 1 à 4 atomes de carbone dans la fraction alkyle, un groupe époxyalkyle contenant 3 à 8 atomes de carbone, de même que les groupements

$$[-X-\overset{\oplus}{N}R^7R^8R^9]\,Z^{\ominus} \quad \text{et} \quad -X-\overset{\overset{O}{\uparrow}}{N}R^7R^8,$$

$R^7$, $R^8$ et $R^9$ sont identiques ou différents et représentent chacun un groupe alkyle contenant 1 à 4 atomes de carbone,

X représente un groupe alkylène contenant 1 à 4 atomes de carbone ou un groupe alkylidène contenant 2 à 4 atomes de carbone,

Y représente un atome d'oxygène ou un atome de soufre, et

Z représente l'anion d'un acide inorganique ou organique.

2. Dérivés N-oxalyle de N-phényl-aminoacides(esters) de formule générale:

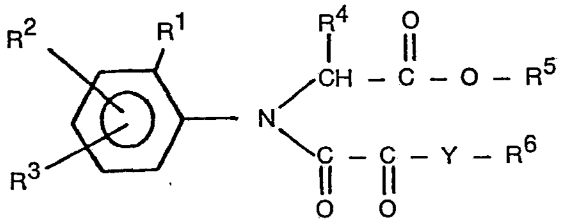

(I)

dans laquelle

$R^1$ représente un atome d'hydrogène, un groupe alkyle contenant 1 à 4 atomes de carbone, ou un atome d'halogène,

$R^2$ représente un atome d'hydrogène ou un groupe alkyle contenant 1 à 4 atomes de carbone,

$R^3$ représente un atome d'hydrogène ou un groupe alkyle contenant 1 à 4 atomes de carbone,

$R^4$ représente un atome d'hydrogène, un groupe alkyle contenant 1 à 4 atomes de carbone ou un groupe phényle éventuellement substitué par un atome d'halogène, par un groupe alkyle contenant 1 ou 2 atomes de carbone ou par un groupe halogénalkyle contenant 1 ou 2 atomes de carbone,

$R^5$ représente un atome d'hydrogène, un groupe alkyle ou un groupe cyanalkyle contenant 1 à 6 atomes de carbone, un groupe alcényle ou un groupe alcinyle contenant 2 à 4 atomes de carbone, un groupe halogénalkyle contenant 1 à 4 atomes de carbone, un groupe cycloalkyle ou un groupe cycloalkylalkyle contenant 3 à 7 atomes de carbone dans la fraction cycloalkyle et 1 à 4 atomes de carbone dans la fraction alkyle, un groupe alcoxyalkyle, un groupe alcoxyalcoxyalkyle, un groupe alcoxycarbonylalkyle, un groupe alkylthioalkyle, un groupe alkylsulfinylalkyle ou un groupe alkylsulfonylalkyle contenant chaque fois 1 à 4 atomes de carbone dans chaque fraction alkyle, un groupe dialkylaminoalkyle contenant 1 à 4 atomes de carbone dans chaque fraction alkyle ou un groupe aryle contenant 6 à 10 atomes de carbone et éventuellement substitué par un atome d'halogène, par un groupe alkyle contenant 1 ou 2 atoms de carbone ou par un groupe halogénalkyle contenant 1 ou 2 atomes de carbone, de même qu'un groupe aralkyle contenant 6 à 10 atomes de carbone dans la fraction aryle et 1 à 4 atomes de carbone dans la fraction alkyle,

$R^6$ représente un groupe alkyle ou un groupe cyanalkyle contenant 1 à 6 atomes de carbone, un groupe alcényle ou un groupe alcinyle contenant 2 à 4 atomes de carbone, un groupe halogénalkyle contenant 1 à 4 atomes de carbone, un groupe cycloalkyle ou un groupe cycloalkylalkyle contenant 3 à 7 atomes de carbone dans la fraction cycloalkyle et 1 à 4 atomes de carbone dans la fraction alkyle, un groupe alcoxyalkyle, un groupe alcoxyalcoxyalkyle, un groupe alcoxycarbonylalkyle, un groupe alkylthioalkyle, un groupe alkylsulfinylalkyle ou un groupe alkylsulfonylalkyle contenant chaque fois 1 à 4 atomes de carbone dans chaque fraction alkyle, un groupe dialkylaminoalkyle contenant 1 à 4 atomes de carbone dans chaque fraction alkyle, un groupe aryle contenant 6 à 10 atomes de carbone et éventuellement substitué par un atome d'halogène, par un groupe alkyle contenant 1 ou 2 atomes de carbone ou par un groupe halogénalkyle contenant 1 ou 2 atomes de carbone, un groupe aralkyle ou un groupe aroxyalkyle contenant 6 à 10 atomes de carbone dans la fraction aryle et 1 à 4 atomes de carbone dans la fraction alkyle, un groupe époxyalkyle contenant 3 à 8 atomes de carbone, de même que les groupements

$$[-X-\overset{\oplus}{N}R^7R^8R^9]\ Z^{\ominus} \text{ et } -X-\overset{\overset{O}{\uparrow}}{N}R^7R^8,$$

$R^7$, $R^8$ et $R^9$ sont identiques ou différents et représentent chacun un groupe alkyle contenant 1 à 4 atomes de carbone,

X représente un groupe alkylène contenant 1 à 4 atomes de carbone ou un groupe alkylidène contenant 2 à 4 atomes de carbone,

Y représente un atome d'oxygène ou un atome de soufre, et

Z représente l'anion d'un acide inorganique ou organique.

3. Procédé de préparation de dérivés N-oxalyle de N-phényl-amino-acides (esters) de formule générale:

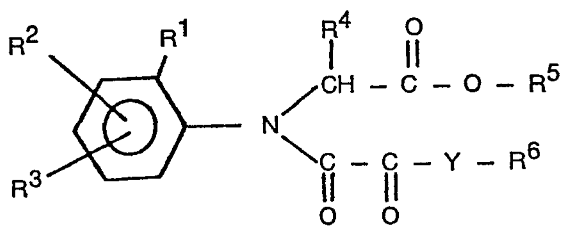

(I)

dans laquelle

$R^1$ représente un atome d'hydrogène, un groupe alkyle contenant 1 à 4 atomes de carbone, ou un atome d'halogène,

$R^2$ représente un atome d'hydrogène ou un groupe alkyle contenant 1 à 4 atomes de carbone,

$R^3$ représente un atome d'hydrogène ou un groupe alkyle contenant 1 à 4 atomes de carbone,

$R^4$ représente un atome d'hydrogène, un groupe alkyle contenant 1 à 4 atomes de carbone ou un groupe phényle éventuellement substitué,

$R^5$ représente un atome d'hydrogène, un groupe alkyle ou un groupe cyanalkyle contenant 1 à 6 atomes de carbone, un groupe alcényle ou un groupe alcinyle contenant 2 à 4 atomes de carbone, un groupe halogénalkyle contenant 1 à 4 atomes de carbone, un groupe cycloalkyle ou un groupe cycloalkylalkyle contenant 3 à 7 atomes de carbone dans la fraction cycloalkyle et 1 à 4 atomes de carbone dans la fraction alkyle, un groupe alcoxyalkyle, un groupe alcoxyalcoxyalkyle, un groupe alcoxycarbonylalkyle, un groupe alkylthioalkyle, un groupe alkylsulfinylalkyle ou un group alkylsulfonylalkyle contenant chaque fois 1 à 4 atomes de carbone dans chaque fraction alkyle, un groupe dialkylamino-alkyle contenant 1 à 4 atomes de carbone dans chaque fraction alkyle ou un

groupe aryle éventuellement substitué et contenant 6 à 10 atomes de carbone, de même qu'un groupe aralkyle contenant 6 à 10 atomes de carbone dans la fraction aryle et 1 à 4 atomes de carbone dans la fraction alkyle,

$R^6$ représente un groupe alkyle ou un groupe cyanalkyle contenant 1 à 6 atomes de carbone, un groupe alcényle ou un groupe alcinyle contenant 2 à 4 atomes de carbone, un groupe halogénalkyle contenant 1 à 4 atomes de carbone, un groupe cycloalkyle ou un groupe cycloalkylalkyle contenant 3 à 7 atomes de carbone dans la fraction cycloalkyle et 1 à 4 atomes de carbone dans la fraction alkyle, un groupe alcoxyalkyle, un groupe alcoxyalcoxyalkyle, un groupe alcoxycarbonylalkyle, un groupe alkylthioalkyle, un groupe alkylsulfinylalkyle ou un groupe alkylsulfonylalkyle contenant chaque fois 1 à 4 atomes de carbone dans chaque fraction alkyle, un groupe dialkylaminoalkyle contenant 1 à 4 atomes de carbone dans chaque fraction alkyle, un groupe aryle contenant 6 à 10 atomes de carbone et éventuellement substitué par un atome d'halogène, par un groupe alkyle contenant 1 ou 2 atomes de carbone ou par un groupe halogénalkyle contenant 1 ou 2 atomes de carbone, un groupe aralkyle ou un groupe aroxyalkyle contenant 6 à 10 atomes de carbone dans la fraction aryle et 1 à 4 atomes de carbone dans la fraction alkyle, un groupe époxyalkyle contenant 3 à 8 atomes de carbone, de même que les groupements

$$[-X-\overset{\oplus}{N}R^7R^8R^9]\,Z^{\ominus} \quad \text{et} \quad -X-\overset{\overset{O}{\uparrow}}{N}R^7R^8$$

$R^7$, $R^8$ et $R^9$ sont identiques ou différents et représentent chacun un groupe alkyle contenant 1 à 4 atomes de carbone,

X représente un groupe alkylène contenant 1 à 4 atomes de carbone ou un groupe alkylidène contenant 2 à 4 atomes de carbone,

Y représente un atome d'oxygène ou un atome de soufre, et

Z représente l'anion d'un acide inorganique ou organique,

caractérise en ce qu'on fait réagir des N-phényl-amino-acides (esters) de formule:

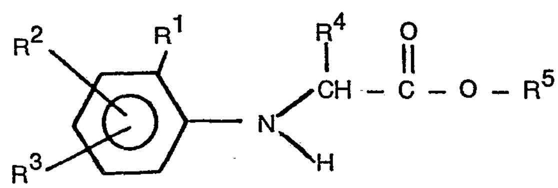

dans laquelle

les radicaux $R^1$ à $R^5$ ont les significations indiquées ci-dessus,

a) avec des esters d'acide chloroglyoxylique de formule:

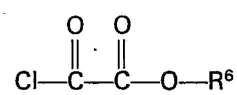

(III)

dans laquelle

$R^6$ a la signification indiquée ci-dessus,

en présence d'un solvant et éventuellement en présence d'un agent fixateur d'acide et/ou en présence d'un catalyseur, ou

b) tout d'abord avec du chlorure d'oxalyle de formule:

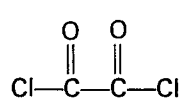

(IV)

en présence d'un solvant et éventuellement en présence d'un agent fixateur d'acide et/ou en présence d'un catalyseur, puis on fait réagir les composés ainsi obtenus de formule

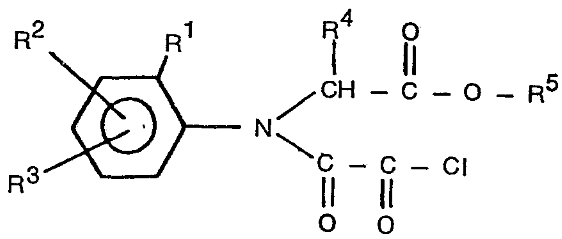

(V)

dans laquelle

les radicaux $R^1$ à $R^5$ ont les significations indiquées dans la revendication 1, avec des alcools ou des mercaptans de formule:

$$H—Y—R^6 \quad (VI)$$

dans laquelle

$R^6$ et Y ont les significations indiquées dans la revendication 1, en présence d'un solvant et éventuellement en présence d'un agent fixateur d'acide et/ou en présence d'un catalyseur.

4. Procédé de préparation de dérivés N-oxalyle de N-phényl-amino-acides (esters) de formule générale:

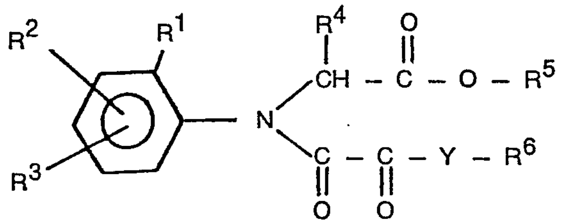

(I)

dans laquelle

$R^1$ représente un atome d'hydrogène, un groupe alkyle contenant 1 à 4 atomes de carbone, ou un atome d'halogène,

$R^2$ représente un atome d'hydrogène ou un groupe alkyle contenant 1 à 4 atomes de carbone,

$R^3$ représente un atome d'hydrogène ou un groupe alkyle contenant 1 à 4 atomes de carbone,

$R^4$ représente un atome d'hydrogène, un groupe alkyle contenant 1 à 4 atomes de carbone ou un groupe phényle éventuellement substitué par un atome d'halogène, par un groupe alkyle contenant 1 ou 2 atomes de carbone ou par un groupe halogénalkyle contenant 1 ou 2 atomes de carbone,

$R^5$ représente un atome d'hydrogène, un groupe alkyle ou un groupe cyanalkyle contenant 1 à 6 atomes de carbone, un groupe alcényle ou un groupe alcinyle contenant 2 à 4 atomes de carbone, un groupe halogénalkyle contenant 1 à 4 atomes de carbone, un groupe cycloalkyle ou un groupe cycloalkylalkyle contenant 3 à 7 atomes de carbone dans la fraction cycloalkyle et 1 à 4 atomes de carbone dans la fraction alkyle, un groupe alcoxyalkyle, un groupe alcoxyalcoxyalkyle, un groupe alcoxycarbonylalkyle, un groupe alkylthioalkyle, un groupe alkylsulfinylalkyle ou un groupe alkylsulfonylalkyle contenant chaque fois 1 à 4 atomes de carbone dans chaque fraction alkyle, un groupe dialkylaminoalkyle contenant 1 à 4 atomes de carbone dans chaque fraction alkyle ou un groupe aryle contenant 6 à 10 atomes de carbone et éventuellement substitué par un atome d'halogène, par un groupe alkyle contenant 1 ou 2 atomes de carbone ou par un groupe halogénalkyle contenant 1 ou 2 atomes de carbone, de même qu'un groupe aralkyle, caractérisé en ce qu'on fait réagir des N-phényl-amino-acides (esters) de formule:

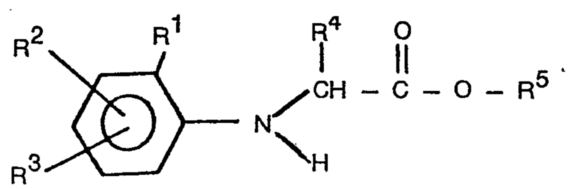

dans laquelle

les radicaux $R^1$ à $R^5$ ont les significations indiquées ci-dessus,

a) avec des esters d'acide chloroglyoxylique de formule:

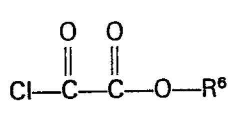

(III)

dans laquelle

$R^6$ a la signification indiquée ci-dessus,

en présence d'un solvant et éventuellement en présence d'un agent fixateur d'acide et/ou en présence d'un catalyseur, ou

b) tout d'abord avec du chlorure d'oxalyle de formule:

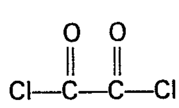

(IV)

en présence d'un solvant et éventuellement en présence d'un agent fixateur d'acide et/ou en présence d'un catalyseur, puis on fait réagir les composés ainsi obtenus de formule

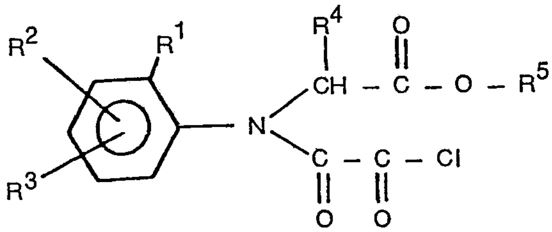

(V)

dans laquelle

les radicaux $R^1$ à $R^5$ ont les significations indiquées ci-dessus,

avec des alcools ou des mercaptans de formule:

$$H—Y—R^6 \qquad (VI)$$

dans laquelle

$R^6$ et Y ont les significations indiquées ci-dessus, en présence d'un solvant et éventuellement en présence d'un agent fixateur d'acide et/ou en présence d'un catalyseur.

5. Agents fongicides, caractérisés en ce qu'ils contiennent au moins un dérivé N-oxalyle de N-phényl-amino-acides (esters) suivant la revendiction 1.

6. Procédé en vue de combattre les champignons, caractérisé en ce qu'on fait agir des dérivés N-oxalyle de N-phényl-amino-acides (esters) suivant les revendications 1 et 2 sur des champignons ou leurs biotopes.

7. Utilisation de dérivés N-oxalyle de N-phényl-amino-acides (esters) suivant les revendications 1 et 2 pour combattre les champignons.

8. Procédé de préparation d'agents fongicides, caractérisé en ce qu'on mélange des dérivés N-oxalyle de N-phényl-amino-acides (esters) suivant les revendications 1 et 2 avec des diluants et/ou des agents tensio-actifs.

**Claims**

1. N-Oxalyl derivatives of N-phenyl-aminoacids and N-phenylaminoacid esters of the general formula

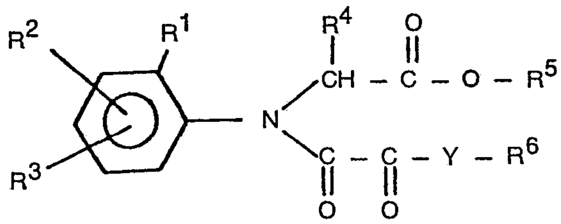

(I)

in which

$R^1$ represents hydrogen, alkyl with 1 to 4 carbon atoms or halogen,

$R^2$ represents hydrogen or alkyl with 1 to 4 carbon atoms,

$R^3$ represents hydrogen or alkyl with 1 to 4 carbon atoms,

$R^4$ represents hydrogen, alkyl with 1 to 4 carbon atoms or optionally substituted phenyl,

$R^5$ represents hydrogen, alkyl and cyanoalkyl with 1 to 6 carbon atoms, alkenyl and alkinyl with 2 to 4 carbon atoms, halogenoalkyl with 1 to 4 carbon atoms, cycloalkyl and cycloalkylalkyl with 3 to 7 carbon atoms in the cycloalkyl part and 1 to 4 carbon atoms in the alkyl part, alkoxyalkyl, alkoxyalkoxyalkyl, alkoxycarbonylalkyl, alkylthioalkyl, alkylsulphinylalkyl and alkylsulphonylalkyl each with 1 to 4 carbon atoms in each alkyl part, dialkylaminoalkyl with 1 to 4 carbon atoms in each alkyl part or optionally substituted aryl with 6 to 10 carbon atoms and aralkyl with 6 to 10 carbon atoms in the aryl part and 1 to 4 carbon atoms in the alkyl part,

$R^6$ represents alkyl and cyanoalkyl with 1 to 6 carbon atoms, alkenyl and alkinyl with 2 to 4 carbon atoms, halogenoalkyl with 1 to 4 carbon atoms, cycloalkyl and cycloalkylalkyl with 3 to 7 carbon atoms in the cycloalkyl part and 1 to 4 carbon atoms in the alkyl part, alkoxyalkyl, alkoxyalkoxyalkyl, alkoxycarbonylalkyl, alkylthioalkyl, alkylsulphinylalkyl and alkylsulphonylalkyl each with 1 to 4 carbon atoms in each alkyl part, dialkylaminoalkyl with 1 to 4 carbon atoms in each alkyl part, optionally substituted aryl with 6 to 10 carbon atoms, aralkyl or aroxyalkyl with 6 to 10 carbon atoms in the aryl part and 1 to 4 carbon atoms in the alkyl part, epoxyalkyl with 3 to 8 carbon atoms as well as the groupings

$$[—X—\overset{\oplus}{N}R^7R^8R^9]\,Z^{\ominus} \quad \text{and} \quad —X—\overset{\overset{O}{\uparrow}}{N}R^7R^8$$

wherein

$R^7$, $R^8$ and $R^9$ are identical or different and represent alkyl with 1 to 4 carbon atoms,

X represents alkylene with 1 to 4 carbon atoms or alkylidene with 2 to 4 carbon atoms,

Y represents oxygen or sulphur and

Z represents the anion of an inorganic or organic acid.

2. N-Oxalyl derivatives of N-phenyl-aminoacids and N-phenyl-aminoacid esters of the general formula

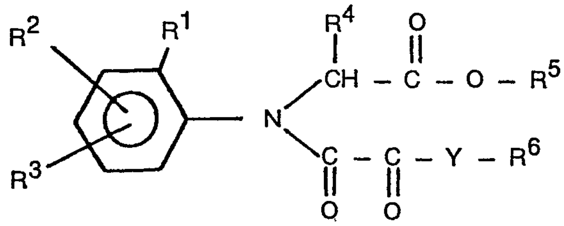

(I)

in which

$R^1$ represents hydrogen, alkyl with 1 to 4 carbon atoms or halogen,

$R^2$ represents hydrogen or alkyl with 1 to 4 carbon atoms,

$R^3$ represents hydrogen or alkyl with 1 to 4 carbon atoms,

$R^4$ represents hydrogen, alkyl with 1 to 4 carbon atoms or phenyl optionally substituted by halogen, alkyl with 1 to 2 carbon atoms and halogenoalkyl with 1 to 2 carbon atoms,

$R^5$ represents hydrogen, alkyl and cyanoalkyl with 1 to 6 carbon atoms, alkenyl and alkinyl with 2 to 4 carbon atoms, halogenoalkyl with 1 to 4 carbon atoms, cycloalkyl and cycloalkylalkyl with 3 to 7 carbon atoms in the cycloalkyl part and 1 to 4 carbon atoms in the alkyl part, alkoxyalkyl, alkoxyalkoxyalkyl, alkoxycarbonylalkyl, alkylthioalkyl, alkylsulphinylalkyl and alkylsulphonylalkyl each with 1 to 4 carbon atoms in each alkyl part, dialkylaminoalkyl with 1 to 4 carbon atoms in each alkyl part or aryl with 6 to 10 carbon atoms which is optionally substituted by halogen, alkyl with 1 to 2 carbon atoms and halogenoalkyl with 1 to 2 carbon atoms, and aralkyl with 6 to 10 carbon atoms in the aryl part and 1 to 4 carbon atoms in the alkyl part,

$R^6$ represents alkyl and cyanoalkyl with 1 to 6 carbon atoms, alkenyl and alkinyl with 2 to 4 carbon atoms, halogenoalkyl with 1 to 4 carbon atoms, cycloalkyl and cycloalkylalkyl with 3 to 7 carbon atoms in the cycloalkyl part and 1 to 4 carbon atoms in the alkyl part, alkoxyalkyl, alkoxyalkoxyalkyl, alkoxycarbonylalkyl, alkylthioalkyl, alkylsulphinylalkyl and alkylsulphonylalkyl each with 1 to 4 carbon atoms in each alkyl part, dialkylaminoalkyl with 1 to 4 carbon atoms in each alkyl part, aryl with 6 to 10 carbon atoms which is optionally substituted by halogen, alkyl with 1 to 2 carbon atoms and halogenoalkyl with 1 to 2 carbon atoms, aralkyl or aroxyalkyl with 6 to 10 carbon atoms in the aryl part and 1 to 4 carbon atoms in the alkyl part, epoxyalkyl with 3 to 8 carbon atoms as well as the groupings

$$[-X-\overset{\oplus}{N}R^7R^8R^9]\,Z^{\ominus} \quad \text{and} \quad -X-\overset{\overset{O}{\uparrow}}{N}R^7R^8$$

wherein

$R^7$, $R^8$ and $R^9$ are identical or different and represent alkyl with 1 to 4 carbon atoms,

X represents alkylene with 1 to 4 carbon atoms or alkylidene with 2 to 4 carbon atoms,

Y represents oxygen or sulphur and

Z represents the anion of an inorganic or organic acid.

3. Process for the preparation of N-oxalyl derivatives of N-phenyl-aminoacids and N-phenyl-aminoacid esters of the general formula

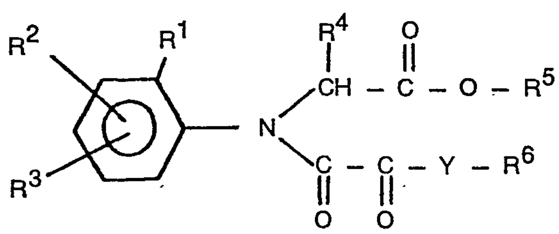

(I)

in which

$R^1$ represents hydrogen, alkyl with 1 to 4 carbon atoms or halogen,

$R^2$ represents hydrogen or alkyl with 1 to 4 carbon atoms,

$R^3$ represents hydrogen or alkyl with 1 to 4 carbon atoms,

$R^4$ represents hydrogen, alkyl with 1 to 4 carbon atoms or optionally substituted phenyl,

$R^5$ represents hydrogen, alkyl and cyanoalkyl with 1 to 6 carbon atoms, alkenyl and alkinyl with 2 to 4 carbon atoms, halogenoalkyl with 1 to 4 carbon atoms, cycloalkyl and cycloalkylalkyl with 3 to 7 carbon atoms in the cycloalkyl part and 1 to 4 carbon atoms in the alkyl part, alkoxyalkyl,

alkoxyalkoxyalkyl, alkoxycarbonylalkyl, alkylthioalkyl, alkylsulphinylalkyl and alkylsulphonylalkyl each with 1 to 4 carbon atoms in each alkyl part, dialkylaminoalkyl with 1 to 4 carbon atoms in each alkyl part or optionally substituted aryl with 6 to 10 carbon atoms and aralkyl with 6 to 10 carbon atoms in the aryl part and 1 to 4 carbon atoms in the alkyl part,

$R^6$ represents alkyl and cyanoalkyl with 1 to 6 carbon atoms, alkenyl and alkinyl with 2 to 4 carbon atoms, halogenoalkyl with 1 to 4 carbon atoms, cycloalkyl and cycloalkylalkyl with 3 to 7 carbon atoms in the cycloalkyl part and 1 to 4 carbon atoms in the alkyl part, alkoxyalkyl, alkoxyalkoxyalkyl, alkoxycarbonylalkyl, alkylthioalkyl, alkylsulphinylalkyl and alkylsulphonylalkyl each with 1 to 4 carbon atoms in each alkyl part, dialkylaminoalkyl with 1 to 4 carbon atoms in each alkyl part, aryl with 6 to 10 carbon atoms which is optionally substituted by halogen, alkyl with 1 to 2 carbon atoms and halogenalkyl with 1 to 2 carbon atoms, aralkyl or aroxyalkyl with 6 to 10 carbon atoms in the aryl part and 1 to 4 carbon atoms in the alkyl part, epoxyalkyl with 3 to 8 carbon atoms as well as the groupings

$$[-X-\overset{\oplus}{N}R^7R^8R^9]\,Z^{\ominus} \quad \text{and} \quad -X-\overset{O}{\overset{\uparrow}{N}}R^7R^8$$

wherein

$R^7$, $R^8$ and $R^9$ are identical or different and represent alkyl with 1 to 4 carbon atoms,

X represents alkylene with 1 to 4 carbon atoms or alkylidene with 2 to 4 carbon atoms,

Y represents oxygen or sulphur and

Z represents the anion of an inorganic or organic acid, characterised in that N-phenylaminoacids or N-phenyl-aminoacid esters of the formula

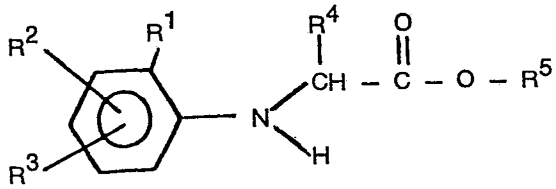

in which

$R^1$ to $R^5$ have the abovementioned meaning,

a) are reacted with chloroglyoxylic acid esters of the formula

$$Cl-\overset{\overset{O}{\|}}{C}-\overset{\overset{O}{\|}}{C}-O-R^6 \qquad (III)$$

in which

$R^6$ has the abovementioned meaning,

in the presence of a solvent and, if appropriate, in the presence of an acid-binding agent and/or in the presence of a catalyst, or

b) are first reacted with oxalyl chloride of the formula

$$Cl-\overset{\overset{O}{\|}}{C}-\overset{\overset{O}{\|}}{C}-Cl \qquad (IV)$$

in the presence of a solvent and, if appropriate, in the presence of an acid-binding agent and/or in the presence of a catalyst, after which the compounds thus produced, of the formula

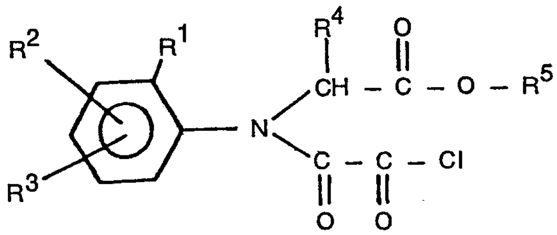

(V)

in which

$R^1$ to $R^5$ have the meaning given in Claim 1 are reacted with alcohols or mercaptans of the formula

$$H-Y-R^6 \qquad (VI)$$

in which

$R^6$ and Y have the meaning given in Claim 1 in the presence of a solvent, and, if appropriate, in the presence of an acid-binding agent and/or in the presence of a catalyst.

4. Process for the preparation of N-oxalyl derivatives of N-phenyl-aminoacids and N-phenyl-aminoacid esters of the general formula

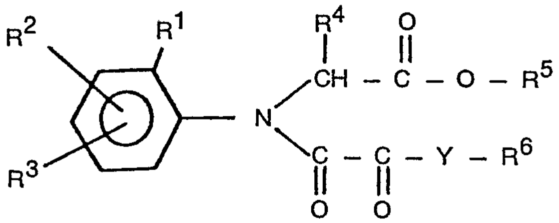

(I)

in which

$R^1$ represents hydrogen, alkyl with 1 to 4 carbon atoms or halogen,

$R^2$ represents hydrogen or alkyl with 1 to 4 carbon atoms,

$R^3$ represents hydrogen or alkyl with 1 to 4 carbon atoms,

$R^4$ represents hydrogen, alkyl with 1 to 4 carbon atoms or phenyl optionally substituted by halogen, alkyl with 1 to 2 carbon atoms and halogenalkyl with 1 to 2 carbon atoms,

$R^5$ represents hydrogen, alkyl and cyanoalkyl with 1 to 6 carbon atoms, alkenyl and alkinyl with 2 to 4 carbon atoms, halogenoalkyl with 1 to 4 carbon atoms, cycloalkyl and cycloalkylalkyl with 3 to 7 carbon atoms in the cycloalkyl part and 1 to 4 carbon atoms in the alkyl part, alkoxyalkyl, alkoxyalkoxyalkyl, alkoxycarbonylalkyl, alkylthioalkyl, alkylsulphinylalkyl and alkylsulphonylalkyl each with 1 to 4 carbon atoms in each alkyl part, dialkylaminoalkyl with 1 to 4 carbon atoms in each alkyl part or aryl with 6 to 10 carbon atoms which is optionally substituted by halogen, alkyl with 1 to 2 carbon atoms and halogenalkyl with 1 to 2 carbon atoms, and aralkyl with 6 to 10 carbon atoms in the aryl part and 1 to 4 carbon atoms in the alkyl part,

$R^6$ represents alkyl and cyanoalkyl with 1 to 6 carbon atoms, alkenyl and alkinyl with 2 to 4 carbon atoms, halogenoalkyl with 1 to 4 carbon atoms, cycloalkyl and cycloalkylalkyl with 3 to 7 carbon atoms in the cycloalkyl part and 1 to 4 carbon atoms in the alkyl part, alkoxyalkyl, alkoxyalkoxyalkyl, alkoxycarbonylalkyl, alkylthioalkyl, alkylsulphinylalkyl and alkylsulphonylalkyl each with 1 to 4 carbon atoms in each alkyl part, dialkylaminoalkyl with 1 to 4 carbon atoms in each alkyl part, aryl with 6 to 10 carbon atoms which is optionally substituted by halogen, alkyl with 1 to 2 carbon atoms and halogenoalkyl with 1 to 2 carbon atoms, aralkyl or aroxyalkyl with 6 to 10 carbon atoms in the aryl part and 1 to 4 carbon atoms in the alkyl part, epoxyalkyl with 3 to 8 carbon atoms as well as the groupings

$$[-X-\overset{\oplus}{N}R^7R^8R^9]\,Z^{\ominus} \quad \text{and} \quad -X-\overset{\overset{O}{\uparrow}}{N}R^7R^8$$

wherein

$R^7$, $R^8$ and $R^9$ are identical or different and represent alkyl with 1 to 4 carbon atoms,

X represents alkylene with 1 to 4 carbon atoms or alkylidene with 2 to 4 carbon atoms,

Y represents oxygen or sulphur and

Z represents the anion of an inorganic or organic acid, characterised in that N-phenylaminoacids or N-phenyl-aminoacid esters of the formula

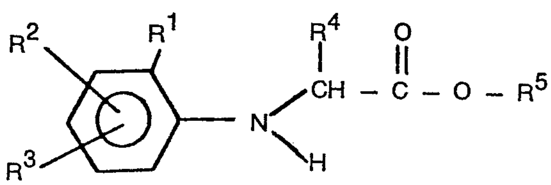

in which

$R^1$ to $R^5$ have the abovementioned meaning,

a) are reacted with chloroglyoxylic acid esters of the formula

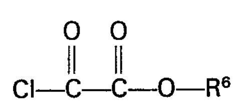

(III)

in which

$R^6$ has the abovementioned meaning,

in the presence of a solvent and, if appropriate, in the presence of an acid-binding agent and/or in the presence of a catalyst, or

b) are first reacted with oxalyl chloride of the formula

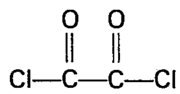 (IV)

in the presence of a solvent and, if appropriate, in the presence of an acid-binding agent and/or in the presence of a catalyst, after which the compounds thus produced, of the formula

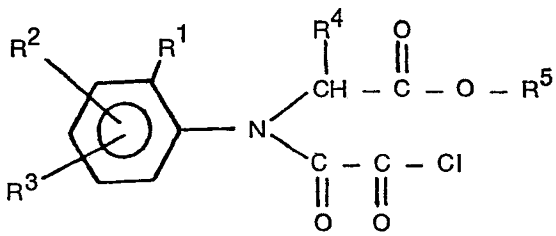 (V)

in which

$R^1$ to $R^5$ have the abovementioned meaning are reacted with alcohols or mercaptans of the formula

$$H—Y—R^6 \quad (VI)$$

in which

$R^6$ and Y have the abovementioned meaning, in the presence of a solvent and, if appropriate, in the presence of an acid-binding agent and/or in the presence of a catalyst.

5. Fungicidal agents, characterised in that they contain at least one N-oxalyl derivative of N-phenyl-aminoacids or N-phenyl-aminoacid esters according to Claim 1.

6. Process for combating fungi, characterised in that N-oxalyl derivatives of N-phenyl-aminoacids or N-phenyl-aminoacid esters according to Claims 1 and 2 are allowed to act on fungi or their habitat.

7. Use of N-oxalyl derivatives of N-phenyl-aminoacids or N-phenyl-aminoacid esters according to Claims 1 and 2 for combating fungi.

8. Process for the preparation of fungicidal agents, characterised in that N-oxalyl derivatives of N-phenyl-aminoacids or N-phenyl-aminoacid esters according to Claims 1 and 2 are mixed with extenders and/or surface-active agents.